(19) Europäisches Patentamt
European Patent Office
Office européen des brevets

(11) EP 1 468 671 B1

(12) FASCICULE DE BREVET EUROPEEN

(45) Date de publication et mention de la délivrance du brevet:
**05.03.2008 Bulletin 2008/10**

(51) Int Cl.:
***A61K 8/29*** *(2006.01)* ***A61K 8/37*** *(2006.01)*
***A61Q 17/04*** *(2006.01)*

(21) Numéro de dépôt: **04290648.7**

(22) Date de dépôt: **10.03.2004**

(54) **Emulsion huile dans eau obtenue par inversion de phase à base de nanopigments d'oxyde métallique et d'un 4,4-diarylbutadiène, procédé de préparation et utilisations**

O/W Emulsion erhältlich durch Phaseninversion enthaltend Nanopigmente von Metalloxiden und ein 4,4-Diarylbutadien, Herstellungsprozess und Verwendung

O/w emulsion obtainable by phase inversion comprising nanopigments of metal oxides and a 4,4'-diarylbutadiene, process for preparation and use

(84) Etats contractants désignés:
**AT BE BG CH CY CZ DE DK EE ES FI FR GB GR HU IE IT LI LU MC NL PL PT RO SE SI SK TR**

(30) Priorité: **14.04.2003 FR 0304645**

(43) Date de publication de la demande:
**20.10.2004 Bulletin 2004/43**

(73) Titulaire: **L'ORÉAL**
**75008 Paris (FR)**

(72) Inventeur: **Candau, Didier**
**91570 Bievres (FR)**

(74) Mandataire: **Miszputen, Laurent**
**L'OREAL - D.I.P.I.**
**25-29 Quai Aulagnier**
**92600 Asnières (FR)**

(56) Documents cités:
**EP-A- 0 668 071**
**EP-A- 0 916 335**
**FR-A- 2 818 128**
**US-A1- 2002 004 034**
**US-B1- 6 191 301**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Jouve, 75001 PARIS (FR)

EP 1 468 671 B1

## Description

L'invention concerne une émulsion huile-dans-eau dans laquelle la taille moyenne des globules qui constituent la phase huileuse de ladite émulsion est comprise entre 100 nm et 1000 nm comprenant des nanopigments minéraux à base d'oxydes métalliques et au moins un filtre UV organique , caractérisée par le fait qu'elle contient au moins un filtre UV organique du type 4,4-diarylbutadiène.

L'invention concerne par ailleurs un procédé de préparation de ces compositions ainsi que leur utilisation dans l'application cosmétique susmentionnée.

Il est bien connu que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions photo toxiques ou photo allergiques. Il est donc souhaitable de filtrer aussi le rayonnement UV-A.

De nombreuses compositions cosmétiques destinées à la photoprotection de la peau ont été proposées à ce jour et l'utilisation de nanopigments d'oxydes métalliques tels que les nanopigments de $TiO_2$ dans les produits solaires est de plus en plus fréquente car ils permettent d'obtenir des indices de protection très élevés en association avec les filtres UV classiques.

Les émulsions huile-dans-eau sont, d'une manière générale, plus appréciées par le consommateur que les émulsions eau-dans-huile, en raison notamment de leur toucher agréable et de leur présentation sous forme de lait ou de crème non gras.

L'un des inconvénients majeurs des compositions antisolaires de type H/E connues à ce jour et plus particulièrement de celles contenant des nanopigments d'oxyde métallique en particulier de titane, est que, une fois appliquées sur la peau sous la forme d'un film, elles engendrent sur cette dernière un effet de blanchissement qui est cosmétiquement indésirable et généralement peu apprécié des utilisateurs. Cet effet est d'autant plus marqué que la concentration en nanopigments dans l'émulsion est élevée et que la distribution après application sur la peau est irrégulière et non homogène. Cette mauvaise répartition des nanopigments que l'on constate à la surface de la peau est souvent liée au fait qu'il existe, au niveau de l'émulsion initiale même (avant application), un manque substantiel d'homogénéité (mauvaise dispersion du pigment dans son support).

Pour remédier à ce problème, il a été proposé des émulsions H/E spécifiques obtenues selon la technique dite "d'inversion de phase" (PIT).

Afin de pouvoir obtenir une bonne protection à la fois dans la gamme des rayons UV-A et dans la gamme UV-B, on utilise dans ce type de composition le plus souvent des filtres organiques actifs dans l'UV-A associés à des filtres organiques actifs dans l'UV-B.

Parmi les filtres UV-A organiques disponibles, une famille de composés particulièrement efficaces dans l'UV-A est l'acide benzène 1,4-di(3-méthylidène-10-camphosulfonique) et ses différents sels, décrite notamment dans les demandes de brevets FR-A-2528420 et FR-A-2639347, ils sont capables en effet d'absorber les rayons ultraviolets de longueur d'ondes comprises entre 280 et 400 nm, avec des maxima d'absorption compris entre 320 et 400 nm, en particulier aux alentours de 345 nm.

Cependant, les émulsions H/E spécifiques obtenues selon la technique dite "d'inversion de phase" contenant des nanopigments et des filtres UVA de ce type perdent leur efficacité en protection UV et notamment en protection UV-A dès lors qu'elles viennent en contact avec l'eau; en effet. ces filtres ont tendance à disparaître à l'eau, par baignade en mer ou en piscine, sous la douche ou lors de la pratique de sports nautiques ; ainsi, ces compositions solaires n'apportent plus la protection initiale recherchée dès lors que le substrat (peau ou cheveu) sur lequel elles ont été appliquées vient en contact avec l'eau.

Ainsi, le besoin subsiste toujours de disposer de compositions antisolaires du type émulsion H/E obtenue selon la technique dite "d'inversion de phase" à base de nanopigments d'oxyde métallique et d'au moins un filtre organique UV-A d'efficacité comparable à celle de l'acide benzène 1,4-di(3-méthylidène-10-camphosulfonique) et ses différents sels qui soient stables dans le temps et résistantes à l'eau (rémanence à l'eau) et dont les performances cosmétiques seraient comparables à celles obtenues avec les émulsions huile/eau classiques.

A la suite d'importantes recherches menées sur la question, il a maintenant été trouvé par la Demanderesse, et ceci de façon tout à fait inattendue et surprenante, qu'il est possible de remédier aux différents inconvénients énumérés ci-dessus, en utilisant un filtre UV-A du type 4,4-diarylbutadiène dans un support du type émulsion H/E obtenue selon la technique dite "d'inversion de phase" à base de nanopigments d'oxyde métallique

Cette découverte est à la base de la présente invention.

Ainsi, conformément à l'un des objets de la présente invention, il est maintenant proposé une émulsion huile-dans-eau susceptible d'être obtenue selon la technique d'inversion de phase dans laquelle la taille moyenne des globules qui constituent la phase huileuse de ladite émulsion est comprise entre 100 nm et 1000 nm et comprenant des nanopigments minéraux à base d'oxydes métalliques et au moins un filtre UV organique, caractérisée par le fait qu'elle contient au moins un filtre UV-A organique du type 4,4-diarylbutadiène.

D'autres caractéristiques, aspects et avantages de l'invention apparaitront à la lecture de la description détaillée qui va suivre.

Les compositions conformes à l'invention contiennent au moins un nanopigment d'oxyde métallique choisi parmi les oxydes de titane, cérium, zirconium, zinc, fer ou leurs mélanges.

On entend par nanopigment un pigment dont la taille moyenne des particules élémentaires est supérieure à 5 nm et inférieure à 100 nm. Selon un mode préféré de réalisation de l'invention, cette taille est de préférence entre 10 nm et 50 nm.

Les nanopigments peuvent être enrobés ou non enrobés.

Les pigments enrobés sont des pigments qui ont subi un ou plusieurs traitements de surface de nature chimique, électronique, mécanochimique et/ou mécanique avec des composés tels que décrits par exemple dans Cosmetics & Toiletries, Février 1990, Vol. 105, p. 53-64, tels que des aminoacides, de la cire d'abeille, des acides gras, des alcools gras, des tensio-actifs anioniques, des lécithines, des sels de sodium, potassium, zinc, fer ou aluminium d'acides gras, des alcoxydes métalliques (de titane ou d'aluminium), du polyéthylène, des silicones, des protéines (collagène, élastine), des alcanolamines, des oxydes de silicium, des oxydes métalliques ou de l'hexamétaphosphate de sodium.

De façon connue, les silicones sont des polymères ou oligomères organo-siliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux des lesquels les atomes de silicium sont reliés entre eux par des atomes d'oxygène (liaison siloxane), des radicaux hydrocarbonés éventuellement substitués étant directement liés par l'intermédiaire d'un atome de carbone sur lesdits atomes de silicium.

Le terme de "silicones" englobe également les silanes nécessaires à leur préparation, en particulier, les alkyl silanes.

Les silicones utilisées pour l'enrobage des nanopigments convenant à la présente invention sont de préférence choisies dans le groupe contenant les alkyl silanes, les polydialkylsiloxanes, et les polyalkylhydrogénosiloxanes. Plus préférentiellement encore, les silicones sont choisies dans le groupe contenant l'octyl triméthyl silane, les polydiméthylsiloxanes et les polyméthylhydro-génosiloxanes.

Bien entendu, les nanopigments d'oxydes métalliques avant leur traitement par des silicones, peuvent avoir été traités par d'autres agents de surface, en particulier par de l'oxyde de cérium, de l'alumine, de la silice, des composés de l'aluminium, des composés du silicium, ou leurs mélanges.

Les pigments enrobés sont plus particulièrement des oxydes de titane enrobés :

- de silice tels que le produit "SUNVEIL" de la société IKEDA,
- de silice et d'oxyde de fer tels que le produit "SUNVEIL F" de la société IKEDA,
- de silice et d'alumine tels que les produits "MICROTITANIUM DIOXIDE MT 500 SA" et"MICROTITANIUM DIOXIDE MT 100 SA" de la société TAYCA, "TIOVEIL" de la société TIOXIDE,
- d'alumine tels que les produits "TIPAQUE TTO-55 (B)" et "TIPAQUE TTO-55 (A)" de la société ISHIHARA, et "UVT 14/4" de la société KEMIRA,
- d'alumine et de stéarate d'aluminium tels que le produit "MICROTITANIUM DIOXIDE MT 100 T, MT 100 TX, MT 100 Z" de la société TAYCA,
- d'alumine et de laurate d'aluminium tel que le produit "MICROTITANIUM DIOXIDE MT 100 S" de la société TAYCA,
- d'oxyde de fer et de stéarate de fer tels que le produit "MICROTITANIUM DIOXIDE MT 100 F" de la société TAYCA,
- d'oxyde de zinc et de stéarate de zinc tels que le produit "BR 351" de la société TAYCA,
- de silice et d'alumine et traités par une silicone tels que les produits "MICROTITANIUM DIOXIDE MT 600 SAS", "MICROTITANIUM DIOXIDE MT 500 SAS" ou "MICROTITANIUM DIOXIDE MT 100 SAS"de la société TAYCA,
- de silice, d'alumine, de stéarate d'aluminium et traités par une silicone tels que le produit "STT-30-DS" de la société TITAN KOGYO,
- d'alumine et traités par une silicone tels que les produits "TIPAQUE TTO-55 (S)" de la société ISHIHARA, ou "UV TITAN M 262" de la société KEMIRA, de triéthanolamine tels que le produit "STT-65-S" de la société TITAN KOGYO,
- d'acide stéarique tels que le produit "TIPAQUE TTO-55 (C)" de la société ISHIHARA,
- d'hexamétaphosphate de sodium tels que le produit "MICROTITANIUM DIOXIDE MT 150 W" de la société TAYCA.

D'autres nanopigments d'oxyde de titane traités avec une silicone sont de préférence le $TiO_2$ traité par l'octyl triméthyl silane et dont la taille moyenne des particules élémentaires est comprise entre 25 et 40 nm tel que celui vendu

sous la dénomination commerciale "T 805" par la société DEGUSSA SILICES, le TiO2 traité par un polydiméthylsiloxane et dont la taille moyenne des particules élémentaires est de 21 nm tel que celui vendu sous la dénomination commerciale "70250 CARDRE UF TiO2SI3" par la société CARDRE, le TiO2 anatase/rutile traité par un polydiméthylhydrogénosiloxane et dont la taille moyenne des particules élémentaires est de 25 nm tel que celui vendu sous la dénomination commerciale "MICRO TITANIUM DIOXYDE USP GRADE HYDROPHOBIC" par la société COLOR TECHNIQUES.

Les nanopigments d'oxyde de titane non enrobés sont par exemple vendus par la société TAYCA sous les dénominations commerciales "MICROTITANIUM DIOXIDE MT 500 B" ou "MICROTITANIUM DIOXIDE MT600 B", par la société DEGUSSA sous la dénomination "P 25", par la société WACKHER sous la dénomination "Oxyde de titane transparent PW", par la société MIYOSHI KASEI sous la dénomination "UFTR", par la société TOMEN sous la dénomination "ITS" et par la société TIOXIDE sous la dénomination "TIOVEIL AQ".

Les nanopigments d'oxyde de zinc non enrobés, sont par exemple

- ceux commercialisés sous la dénomination "Z-COTE" par la société SUNSMART ;
- ceux commercialisés sous la dénomination "NANOX" par la société ELEMENTIS ;
- ceux commercialisés sous la dénomination "NANOGARD WCD 2025" par la société NANOPHASE TECHNOLOGIES ;

Les nanopigments d'oxyde de zinc enrobés sont par exemple

- ceux commercialisés sous la dénomination "OXIDE ZINC CS-5" par la société Toshibi (ZnO enrobé par polymethylhydrogenesiloxane) ;
- ceux commercialisés sous la dénomination "NANOGARD ZINC OXIDE FN" par la société NANOPHASE TECHNOLOGIES (en dispersion à 40% dans le Finsolv TN, benzoate d'alcools en $C_{12}$-$C_{15}$) ;
- ceux commercialisés sous la dénomination "DAITOPERSION ZN-30" et "DAITOPERSION ZN-50" par la société Daito (dispersions dans cyclopolyméthylsiloxane /polydiméthylsiloxane oxyéthyléné, contenant 30% ou 50% de nano-oxydes de zinc enrobés par la silice et le polyméthylhydrogènesiloxane) ;
- ceux commercialisés sous la dénomination "NFD ULTRAFINE ZNO" par la société Daikin (ZnO enrobé par phosphate de perfluoroalkyle et copolymère à base de perfluoroalkyléthyle en dispersion dans du cyclopentasiloxane) ;
- ceux commercialisés sous la dénomination "SPD-Z1" par la société Shin-Etsu (ZnO enrobé par polymère acrylique greffé silicone, dispersé dans cyclodiméthylsiloxane) ;
- ceux commercialisés sous la dénomination "ESCALOL Z100" par la société ISP (ZnO traité alumine et dispersé dans le mélange methoxycinnamate d'ethylhexyle / copolymère PVP-hexadecene / methicone) ;
- ceux commercialisés sous la dénomination "FUJI ZNO-SMS-10" par la société Fuji Pigment (ZnO enrobé silice et polymethylsilsesquioxane) ;
- ceux commercialisés sous la dénomination "NANOX GEL TN" par la société Elementis (ZnO dispersé à 55% dans du benzoate d'alcools en $C_{12}$-$C_{15}$ avec polycondensat d'acide hydroxystéarique).

Les nanopigments d'oxyde de cérium non enrobé est vendu sous la dénomination "COLLOIDAL CERIUM OXIDE" par la société RHONE POULENC.

Les nanopigments d'oxyde de fer non enrobés sont par exemple vendus par la société ARNAUD sous les dénominations "NANOGARD WCD 2002 (FE 45B)", "NANOGARD IRON FE 45 BL AQ", "NANOGARD FE 45R AQ, "NANOGARD WCD 2006 (FE 45R)", ou par la société MITSUBISHI sous la dénomination "TY-220".

Les nanopigments d'oxyde de fer enrobés sont par exemple vendus par la société ARNAUD sous les dénominations "NANOGARD WCD 2008 (FE 45B FN)", "NANOGARD WCD 2009 (FE 45B 556)", "NANOGARD FE 45 BL 345", "NANOGARD FE 45 BL", ou par la société BASF sous la dénomination "OXYDE DE FER TRANSPARENT".

On peut également citer les mélanges d'oxydes métalliques, notamment de dioxyde de titane et de dioxyde de cérium, dont le mélange équipondéral de dioxyde de titane et de dioxyde de cérium enrobés de silice, vendu par la société IKEDA sous la dénomination "SUNVEIL A", ainsi que le mélange de dioxyde de titane et de dioxyde de zinc enrobé d'alumine, de silice et de silicone tel que le produit "M 261" vendu par la société KEMIRA ou enrobé d'alumine, de silice et de glycérine tel que le produit "M 211" vendu par la société KEMIRA.

Selon l'invention, les nanopigments d'oxyde de titane, enrobés ou non enrobés, sont particulièrement préférés.

L'oxyde de titane peut se présenter sous forme rutile, anatase ou amorphe, mais de préférence sous forme rutile et/ou anatase et/ou sous une forme amorphe ou substantiellement amorphe.

Selon leur caractère lipophile, ou au contraire hydrophile, plus ou moins prononcé, les nanopigments pourront être présents soit dans la phase grasse de l'émulsion, soit dans la phase aqueuse, ou bien même encore dans les deux phases à la fois.

Les nanopigments conformes à l'invention représente en général de 0,5 à 40 %, de préférence de 1 à 30 %, du poids total de l'émulsion.

Les composés 4,4-diarylbutadiènes conformes à l'invention sont choisis de préférence parmi ceux répondant à la formule (1) suivante :

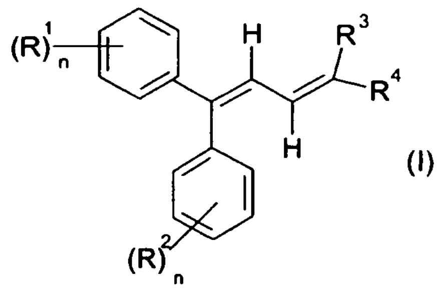

(I)

dans laquelle le système diène est de configuration Z,Z ; Z,E ; E,Z ou E,E ou des mélanges desdites configurations et où :

- $R^1$ et $R^2$, identiques ou différents, désignent hydrogène, un radical alkyle en $C_1$-$C_{20}$; un radical alcényle en $C_2$-$C_{10}$ ; un radical alcoxy en $C_1$-$C_{12}$ ; un radical cycloalkyle en $C_3$-$C_{10}$ ; un radical cycloalcényle en $C_3$-$C_{10}$; un radical alcoxycarbonyle en $C_1$-$C_{20}$ ; un radical monoalkylamino en $C_1$-$C_{12}$ ; un radical dialkylamino en $C_1$-$C_{12}$ ; un aryle ; un hétéroaryle ou un substituant hydrosolubilisant choisi parmi un reste carboxylate, sulfonate ou un reste ammonium ;
- $R^3$ désigne un groupe $COOR^5$ ; $COR^5$; $CONR^5R^6$ ; CN ; $O=S(-R^5)=O$ ; $O=S(-OR^5)=O$ ; $R^7O-P-(-OR^8)=O$; un radical alkyle en $C_1$-$C_{20}$ ; un radical alcényle en $C_2$- $C_{10}$ ; un radical cycloalkyle en $C_3$-$C_{10}$ ; un radical bicycloalkyle en $C_7$-$C_{10}$ ; un radical cycloalcényle en $C_3$-$C_{10}$ ; un radical bicycloalcényle en $C_7$-$C_{10}$ ; un aryle en $C_6$-$C_{18}$ éventuellement substitué ; un hétéroaryle en $C_3$-$C_7$ éventuellement substitué;
- $R^4$ désigne un groupe $COOR^6$; $COR^6$ ; $CONR^5R^6$ ; CN ; $O=S(-R^6)=O$ ; $O=S(-OR^6)=O$ ; $R^7O-P-(-OR^8)=O$; un radical alkyle en $C_1$-$C_{20}$ ; un radical alcényle en $C_2$- $C_{10}$ ; un radical cycloalkyle en $C_3$-$C_{10}$ ; un radical bicycloalkyle en $C_7$-$C_{10}$ ; un radical cycloalcényle en $C_3$-$C_{10}$ ; un radical bicycloalcényle en $C_7$-$C_{10}$ ; un aryle en $C_6$-$C_{18}$ éventuellement substitué ; un hétéroaryle en $C_3$-$C_7$ éventuellement substitué;
- les radicaux $R^5$ à $R^8$, identiques ou différents, désignent hydrogène ; un radical alkyle en $C_1$-$C_{20}$ ; un radical alcényle en $C_2$-$C_{10}$ ; un radical cycloalkyle en $C_3$-$C_{10}$ ; un radical bicycloalkyle en $C_7$-$C_{10}$ ; un radical bicycloalcényle en $C_3$-$C_{10}$ ; un radical cycloalcényle en $C_7$-$C_{10}$ ; un aryle éventuellement substitué ; un hétéroaryle éventuellement substitué ;
- n varie de 1 à 3 ;

les radicaux $R^3$ à $R^8$ peuvent former entre eux avec les atomes de carbone auxquels ils sont liés, un noyau en $C_5$-$C_6$ pouvant être condensé.

Comme radicaux alkyle en $C_1$-$C_{20}$, on peut citer par exemple : méthyle, éthyle, n-propyle, 1-méthyléthyle, n-butyle, 1-méthylpropyle, 2-méthylpropyle, 1,1-diméthyléthyle, n-pentyle, 1-méthylbutyle, 2-méthylbutyle, 3-méthylbutyle, 2,2-diméthylpropyle, 1-éthylpropyle, n-hexyle, 1,1-diméthylpropyle, 1,2-diméthylpropyle, 1-méthylpentyle, 2-méthylpentyle, 3-méthylpentyle, 4-méthylpentyle, 1,1-diméthylbutyle, 1,2-diméthylbutyle, 1,3-diméthylbutyle, 2,2-diméthylbutyle, 2,3-diméthylbutyle, 3,3-diméthylbutyle, 1-éthylbutyle, 2-éthylbutyle, 1,2,2-triméthylpropyle, 1-éthyl-1-méthylpropyle, 1-éthyl-2-méthylpropyle, n-heptyle, n-octyle, n-nonyle, n-décyle, n-undécyle, n-dodécyle, n-tridécyle, n-tétradécyle, n-pentadécyle, n-hexadécyle, n-heptadécyle, n-octadécyle, n-nonadécyle ou n-eicosyle.

Comme groupes alcènyle en $C_2$-$C_{10}$, on peut citer par exemple : éthènyle, n-propènyle, 1-méthyléthènyle, n-butènyle, 1-méthylpropènyle, 2-méthylpropènyle, 1,1-diméthyléthènyle, n-pentènyle, 1-méthylbutènyle, 2-méthylbutènyle, 3-méthylbutènyle, 2,2-diméthylpropènyle, 1-éthylpropènyle, n-hexènyle, 1,1-diméthylpropènyle, 1,2-diméthylpropènyle, 1-méthylpentènyle, 2-méthylpentènyle, 3-méthylpentènyle, 4-méthylpentènyle, 1,1-diméthylbutènyle, 1,2-diméthylbutènyle, 1,3-diméthylbutènyle, 2,2-diméthylbutènyle, 2,3-diméthylbutènyle, 3,3-diméthylbutènyle, 1-éthylbutènyle, 2-éthylbutènyle, 1,1,2-triméthytpropènyle, 1,2,2-triméthylpropènyle, 1-éthyl-1-méthylpropènyle, 1-éthyl-2-méthylpropènyle, n-heptènyle, n-octènyle, n-nonènyle, n-décènyle.

Comme radicaux alcoxy en $C_1$-$C_{12}$, on peut citer : méthoxy, n-propoxy, 1-méthylpropoxy, 1-méthyléthoxy, n-pentoxy, 3-méthylbutoxy, 2,2-diméthylpropoxy, 1-méthyl-1-éthylpropoxy, octoxy, éthoxy, n-propoxy, n-butoxy, 2-méthylpropoxy, 1,1-diméthylpropoxy, hexoxy, heptoxy, 2-éthylhexoxy.

Comme radicaux alcoxycarbonyle en $C_1$-$C_{20}$, on peut citer les esters des alcools en $C_1$-$C_{20}$.

Comme radicaux monoalkylamino ou dialkylamino en $C_1$-$C_{12}$, on peut citer ceux dont le ou les radicaux alkyle sont choisis parmi méthyle, n-propyle, 2-méthylpropyle, 1,1-diméthyléthyle, hexyle, heptyle, 2-éthylhexyle, isopropyle, 1-méthylpropyle, n-pentyle, 3-méthylbutyle, 2,2-diméthylpropyle, 1-méthyl-1-éthylpropyle, octyle.

Comme radicaux cycloalkyles en $C_3$-$C_{10}$, on peut citer par exemple : cyclopropyle, cyclobutyle, cyclopentyle, cyclohexyle, cycloheptyle, 1-méthylcyclopropyle, 1-éthylcyclopropyle, 1-propylcyclopropyle, 1-butylcyclopropyle, 1-pentylcyclopropyle, 1-méthyl-1-butylcyclopropyle, 1,2-diméthylcyclypropyle, 1-méthyl-2-éthylcyclopropyle, cyclooctyle, cyclononyle ou cyclodécyle.

Comme radicaux cycloalcènyles en $C_3$-$C_{10}$ ayant une ou plusieurs doubles liaisons, on peut citer : cyclobutènyle, cyclopentènyle, cyclopentadiènyle, cyclohexènyle, 1,3-cyclohexadiènyle, 1,4-cyclohexadiènyle, cycloheptènyle, cycloheptatriènyle, cyclooctènyle, 1,5-cyclooctadiènyle, cyclooctétraènyle, cyclononènyle ou cyclodécènyle.

Les radicaux cycloalkyles ou cycloalcényles peuvent comporter un ou plusieurs substituants (de préférence de 1 à 3) choisis par exemple parmi les halogènes comme chlore, fluor ou brome ; cyano ; nitro ; amino ; $C_1$-$C_4$-alkylamino ; $C_1$-$C_4$ dialkylamino ; $C_1$-$C_4$alkyle ; $C_1$-$C_4$-alcoxy ; hydroxy ; ils peuvent également comporter de 1 à 3 hétéroatomes comme souffre, oxygène ou azote dont les valences libres peuvent être saturées par un hydrogène ou un radical alkyle en $C_1$-$C_4$.

Les groupes bicycloalkyles ou bicycloalcényles sont choisis par exemple parmi les terpènes bicycliques comme les dérivés de pinane, de bornane, de pinène ou de camphre ou d'adamantane.

Les groupes aryles sont de préférence choisis parmi les cycles phényle ou naphtyle, lesquels pouvant comporter un ou plusieurs substituants (de préférence de 1 à 3) chois par exemple parmi halogène comme chlore, fluor ou brome ; cyano ; nitro ; amino ; $C_1$-$C_4$-alkylamino ; $C_1$-$C_4$ dialkylamino ; $C_1$-$C_4$alkyle ; $C_1$-$C_4$-alcoxy ; hydroxy. On préfère plus particulièrement phényle, méthoxyphényle , naphtyle, thienyle.

Les groupes hétéroaryles comportent en général un ou plusieurs hétéroatomes choisis parmi souffre, oxygène ou azote.

Les groupes hydrosolubilisants sont par exemple des restes carboxy, sulfoxy et plus particulièrement leurs sels avec des cations physiologiquement acceptables comme les sels de métaux alcalins ou les sels de trialkylammonium comme les sels de tri(hydroxyalkyl)ammonium ou de 2-méthylpropan-1-ol-2-ammonium. On peut également citer les groupes ammonium comme les alkylammoniums et leurs formes salifiées avec des anions physiologiquement acceptables.

Les composés de formule (1) sont connus en eux-mêmes et leurs structures et leurs synthèses sont décrites dans les demandes de brevet DE19755649 , EP916335, EP1133980 et EP1133981.

A titre d'exemple de composé de formule (1), on peut citer les composés suivants :

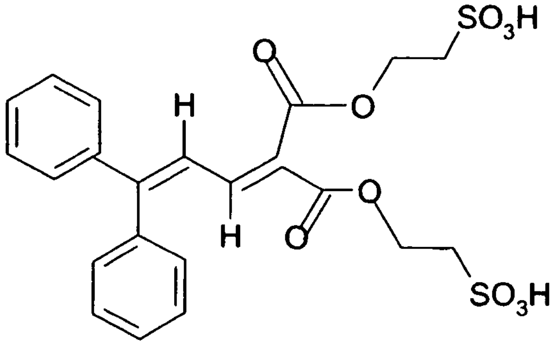

(composé a)

**8**

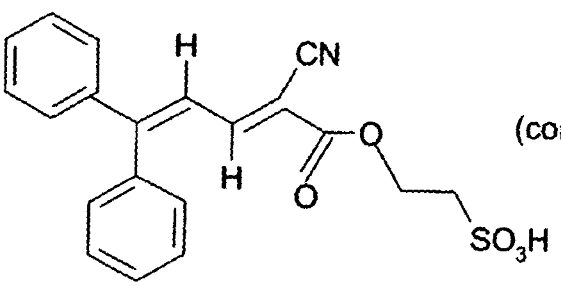

(composé b)

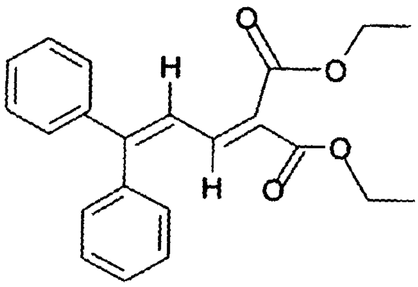

(composé c)

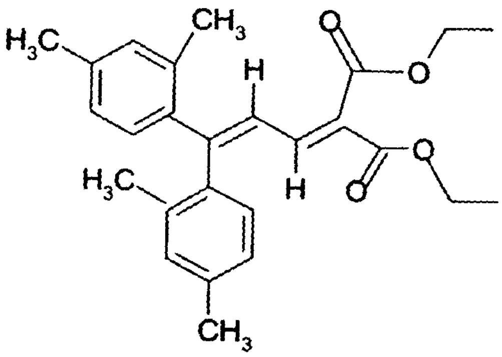

(composé d)

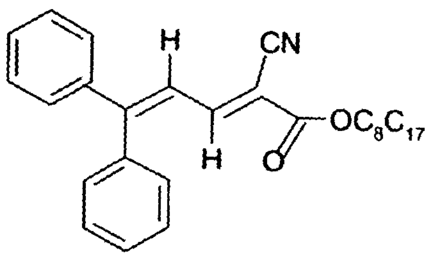

(composé e)

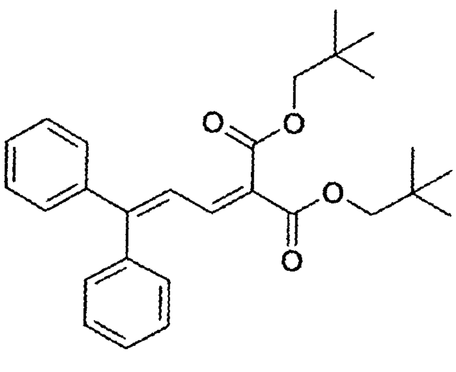

(composé f)

Les composés de formule (1) préférentiels sont ceux pour lesquels

- n = 1 ou 2 ;
- $R^1$ et $R^2$, identiques ou différents, désignent hydrogène, un radical alkyle en $C_1$-$C_{20,}$; un radical alcoxy en $C_1$-$C_{12}$ ; un radical monoalkylamino en $C_1$-$C_{12}$ ; un radical dialkylamino en $C_1$-$C_{12}$ ; un substituant hydrosolubilisant choisi

parmi un groupe carboxylate, un groupe sulfonate ou un reste ammonium ;

- $R^3$ désigne un groupe $COOR^5$; $COR^5$; $CONR^5R^6$ ; un radical alkyle en $C_1$-$C_{20}$ ; un radical cycloalkyle en $C_3$-$C_{10}$ ; un radical cycloalcényle en $C_3$-$C_{10}$ un radical bicycloalkyle en $C_7$-$C_{10}$ ; phényle, naphtyle ou thienyle éventuellement substitué ;
- $R^4$ désigne un groupe $COOR^6$ ; $COR^6$ ; $CONR^5R^6$; un radical alkyle en $C_1$-$C_{20}$ ; un radical cycloalkyle en $C_3$-$C_6$ ; un radical cycloalcényle en $C_3$-$C_{10}$ ; un radical bicycloalkyle en $C_7$-$C_{10}$ ; phényle, naphtyle ou thienyle éventuellement substitué ;
- les radicaux $R^5$ et $R^6$, identiques ou différents, désignent hydrogène ; un radical alkyle en $C_1$-$C_{12}$ ; un radical cycloalkyle en $C_3$-$C_{10}$ ; un radical cycloalcényle en $C_3$-$C_{10}$ ; un radical bicycloalkyle en $C_7$-$C_{10}$ ; un radical bicycloalcényle en $C_3$-$C_{10}$ ; phényle ou naphtyle éventuellement substitué.

Parmi ces composés, on préfère plus particulièrement ceux pour lesquels

- $R^1$ et $R^2$, identiques ou différents, désignent hydrogène, un radical alkyle en $C_1$-$C_{20}$ ; un radical alcoxy en $C_1$-$C_{20}$ ; un substituant hydrosolubilisant choisi parmi un groupe carboxylate, un groupe sulfonate ou un reste ammonium ;
- $R^3$ désigne un groupe $COOR^5$ ; $COR^5$ ; $CONR^5R^6$ ;
- $R^4$ désigne un groupe $COOR^6$ ; $COR^6$ ; $CONR^5R^6$ ;
- les radicaux $R^5$ et $R^6$, identiques ou différents, désignent hydrogène ; un radical alkyle en $C_1$-$C_{12}$ ; un radical cycloalkyle en $C_3$-$C_6$ ; un radical cycloalcényle en $C_3$-$C_{10}$ ; un radical bicycloalkyle en $C_7$-$C_{10}$ ; un radical bicycloalcényle en $C_3$-$C_{10}$ ; phényle ou naphtyle éventuellement substitué.

Selon un mode particulièrement préféré, les composés de formule (1) sont choisis parmi ceux de formule (1') suivante :

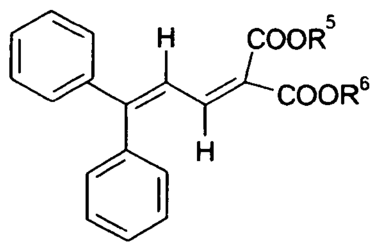

(I')

où les radicaux $R^5$ et $R^6$, identiques ou différents, désignent hydrogène ; un radical alkyle en $C_1$-$C_{20}$ ; un radical cycloalkyle en $C_3$-$C_6$ ; un radical cycloalcényle en $C_3$-$C_{10}$.

Parmi ces composés de formule (1'), on retiendra plus particulièrement le 1,1-dicarboxy (2'2'-diméthyl-propyl)-4,4-diphénylbutadiène de structure :

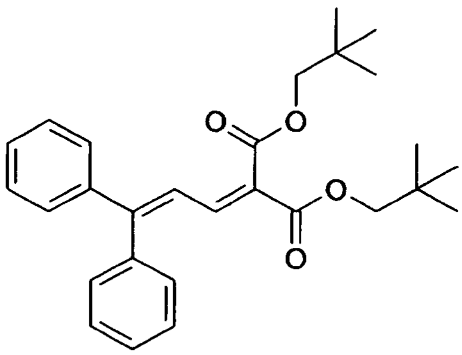

(composé f)

Une autre famille de 4,4-diarylbutadiène pouvant être utilisée dans les émulsions selon l'invention sont ceux répondant à la formule (II) suivante :

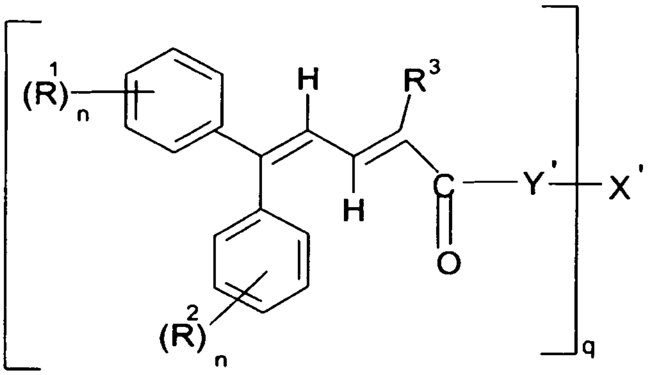

(II)

dans laquelle le système diène est de configuration Z,Z ; Z,E ; E,Z ou E,E ou des mélanges desdites configurations et où :

- $R^1$, $R^2$, $R^3$ et n ont les mêmes significations indiquées dans la formule (1) précédente ;
- Y' désigne un groupe -O- ou -$NR^9$-
- $R^9$ désigne hydrogène ; un radical alkyle en $C_1$-$C_{20}$, linéaire ou ramifié ; un radical alcényle en $C_2$- $C_{10}$ ; un radical cycloalkyle en $C_3$-$C_{10}$ ; un radical bicycloalkyle en $C_7$-$C_{10}$ ; un radical cycloalcényle en $C_3$-$C_{10}$ ; ; un radical bicycloalcényle en $C_7$-$C_{10}$ ; un aryle ; un hétéroaryle;
- X' désigne un reste de polyol linéaire ou ramifié, aliphatique ou cycloaliphatique comprenant de 2 à10 groupes hydroxy et de valence q ; la chaîne carbonée dudit reste pouvant être interrompue par un ou plusieurs atomes de souffre ou d'oxygène ; un ou plusieurs groupes imines ; un ou plusieurs alkylimino en $C_1$-$C_4$ ;
- q varie de 2 à 10.

X' est un reste polyol en $C_2$-$C_{20}$ contenant de 2 à 10 groupes hydroxyles et notamment :

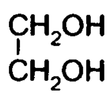

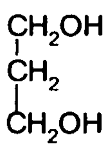

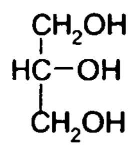

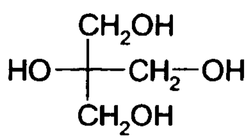

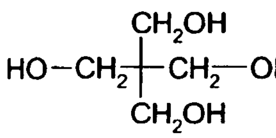

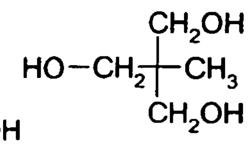

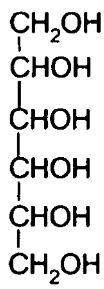

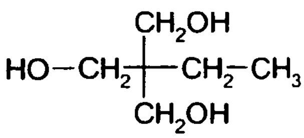

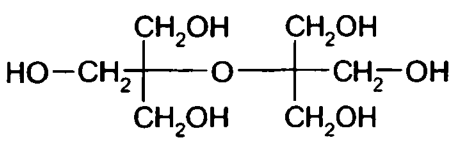

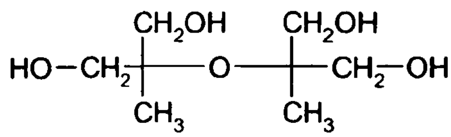

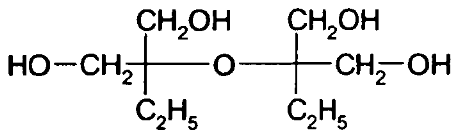

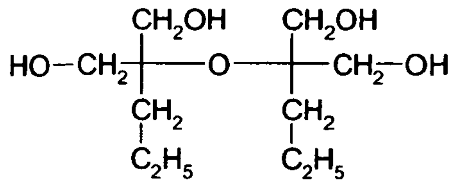

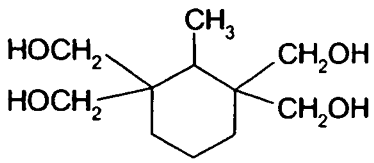

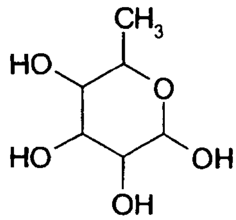

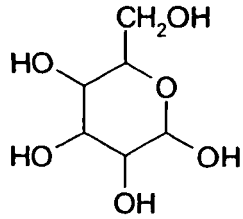

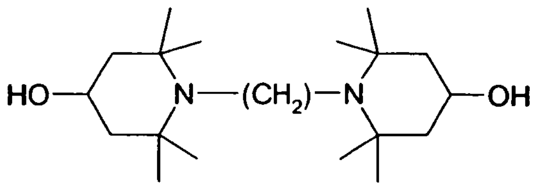

Les composés plus préférentiels de formule (II) sont ceux pour lesquels :

- $R^1$ et $R^2$, identiques ou différents, désignent hydrogène, un radical alkyle en $C_1$-$C_{12}$ ; un radical alcoxy en $C_1$-$C_8$ ; un substituant hydrosolubilisant choisi parmi un groupe carboxylate, un groupe sulfonate ou un reste ammonium ;
- $R^3$ désigne un groupe $COOR^5$ ; $CONR^5R^6$ ; CN ; un radical cycloalkyle en $C_3$-$C_{10}$ ; un radical bicycloalkyle en $C^7$-$C_{10}$ ;
- $R^5$ et $R^6$ , identiques ou différents, désignent un radical alkyle en $C_1$-$C_{20}$, linéaire ou ramifié ; un radical cycloalkyle en $C_3$-$C_{10}$ ; un radical bicycloalkyle en $C_7$-$C_{10}$ ; naphtyle ou phényle éventuellement substitué;
- X' désigne un reste de polyol en $C_2$-$C_{20}$ comprenant de 2 à 6 groupes hydroxy et plus particulièrement de 2 à 4.

Les composés encore plus préférentiels de formule (II) sont ceux pour lesquels :

- X' désigne un reste d'éthanol ou de pentaerythritol.

Les composés de formule (II) encore plus particulièrement préférés sont choisis parmi

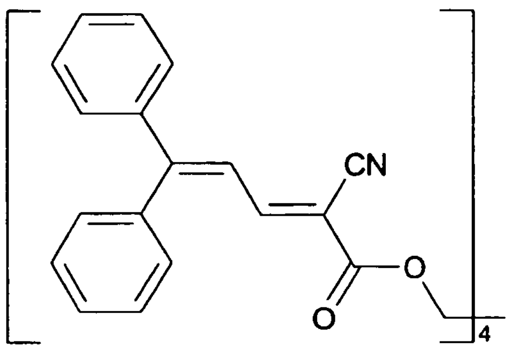

(composé g)

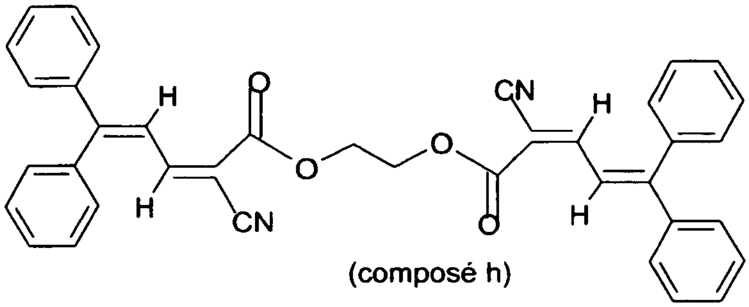

(composé h)

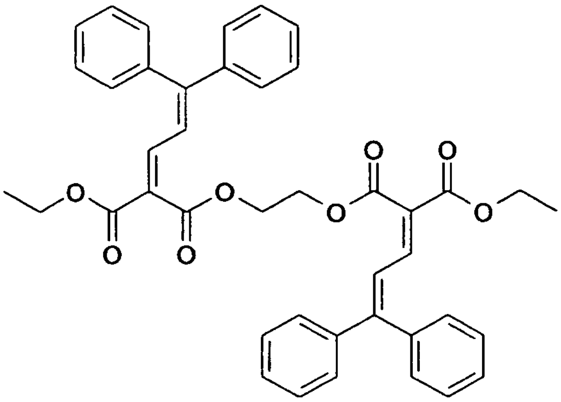

(composé i)

:

Les composés de formule (II) tels que définis ci-dessus sont connus en eux-mêmes et leurs structures et leurs synthèses sont décrites dans la demande de brevet EP-A-1008586.

Les composés 4,4-diarylbutadiène sont présents de préférence dans la composition dans des proportions allant de 0,1 % à 20% en poids, plus préférentiellement de 1 à 10% en poids par rapport au poids total de la composition.

La nature de la phase grasse rentrant dans la composition des émulsions selon l'invention n'est pas critique et elle peut ainsi être constituée par tous les composés qui sont déja connus de façon générale comme convenant pour la fabrication d'émulsions de type huile dans eau. En particulier, ces composés peuvent être choisis, seuls ou en mélanges, parmi les différents corps gras, les huiles d'origine végétale, animale ou minérale, les cires naturelles ou synthétiques, et analogues.

Parmi les huiles pouvant rentrer dans la composition de la phase grasse, on peut notamment citer :

- les huiles minérales telles que l'huile de paraffine et l'huile de vaseline,
- les huiles d'origine animale telles que le perhydrosqualène,
- les huiles d'origine végétale telles que l'huile d'amande douce, l'huile d'avocat, l'huile de ricin, l'huile d'olive, l'huile de jojoba, l'huile de sésame, l'huile d'arachide, l'huile de pépins de raisin, l'huile de colza, l'huile de coprah, l'huile de noisette, le beurre de karité, l'huile de palme, l'huile de noyau d'abricot, l'huile de calophyllum, l'huile de son de riz, l'huile de germes de maïs, l'huile de germes de blé, l'huile de soja, l'huile de tournesol, l'huile d'onagre, l'huile de carthame, l'huile de passiflore et l'huile de seigle,
- les huiles synthétiques telles que l'huile de purcellin, le myristate de butyle, le myristate d'isopropyle, le myristate de cétyle, le palmitate d'isopropyle, l'adipate d'isopropyle, l'adipate d'éthylhéxyle, le stéarate de butyle, le stéarate d'héxadécyle, le stéarate d'isopropyle, le stéarate d'octyle, le stéarate d'isocétyle, l'oléate de décyle, le laurate d'héxyle, le dicaprylate de propylène glycol et les esters dérivés d'acide lanolique tels que le lanolate d'isopropyle, le lanolate d'isocétyle, les isoparaffines et les poly-$\alpha$-oléfines.

Comme autres huiles utilisables dans les émulsions selon l'invention, on peut encore citer les benzoates d'alcools gras en C12-C15 (Finsolv TN de FINETEX), les alcools gras tels que l'alcool laurique, cétylique, myristique, stéarique, palmitique, oléique ainsi que le 2-octyldodécanol, les acétylglycérides, les octanoates et décanoates d'alcools et de polyalcools tels que ceux de glycol et de glycérol, les ricinoléates d'alcools et de polyalcools tels que ceux de cétyle, les triglycérides d'acides gras tels que les triglycérides caprylique/caprique, les triglycérides d'acides gras saturés en C10-C18, les huiles fluorées et perfluorées, la lanoline, la lanoline hydrogénée, la lanoline acétylée et enfin les huiles de silicones, volatiles ou non.

Bien entendu, la phase grasse peut également contenir un ou plusieurs adjuvants cosmétiques lipophiles classiques, notamment ceux qui sont déjà utilisés de manière habituelle dans la fabrication et l'obtention des compositions cosmétiques antisolaires.

Selon une caractéristique essentielle de la présente invention, la taille moyenne des particules liquides (ou globules) de phase grasse au sein de la phase aqueuse dispersante doit être comprise dans des limites bien particulières, à savoir entre 100 nm et 1000 nm. De préférence, cette taille moyenne est comprise entre 100 nm et 500 nm. Encore plus préférentiellement, la distribution en taille des globules huileux est telle que la plupart desdits globules (i.e au moins 90% en nombre) présentent une taille comprise entre les bornes indiquées ci-avant.

De manière classique, la phase aqueuse dispersante peut être constituée par de l'eau, ou un mélange d'eau et d'alcool(s) polyhydrique(s) comme par exemple glycérol, propylèneglycol et sorbitol, ou bien encore un mélange d'eau et d'alcool(s) inférieur(s) hydrosoluble(s) tels que éthanol, isopropanol ou butanol (solution hydroalcoolique), et elle peut bien entendu en outre contenir des adjuvents cosmétiques classiques hydrosolubles.

Les émulsions conformes à l'invention contiennent en outre généralement des tensio-actifs ou émulsionnants particuliers dont l'emploi a été rendu nécessaire pour la préparation et l'obtention de l'émulsion ultrafine. Ce point sera détaillé par la suite. Elles peuvent en outre contenir des co-émulsionnants spécifiques dont le rôle est, lors de la préparation de l'émulsion, de diminuer de manière substantielle la quantité d'agents tensio-actifs nécessaire à la réalisation de l'émulsion.

A titre indicatif, les formulations antisolaires conformes à l'invention présentent généralement les compositions suivantes :

(i) phase aqueuse : de 50 à 95 % en poids, de préférence de 70 à 90 % en poids, par rapport à l'ensemble de la formulation,
(ii) phase huileuse : de 5 à 50 % en poids, de préférence de 10 à 30 % en poids, par rapport à l'ensemble de la formulation,
(iv) (co)émulsionnant(s) : de 0,5 à 20 % en poids, de préférence de 2 à 10% en poids, par rapport à l'ensemble de la formulation.

Le procédé préféré de préparation des compositions selon l'invention, lui-même constitutif d'un second objet de la présente invention, va maintenant être développé. Le procédé d'obtention de ces émulsions repose sur la technique de fabrication par inversion de phase. Cette technique est, dans son principe, bien connue et notamment décrite dans l'article "Phase Inversion Emusification", par Th Förster et al, paru dans Cosmetics & Toiletries, vol. 106, Decembre 1991, pp 49-52. Son principe est le suivant : (i) On mélange, en présence d'un système émulsionnant convenable et d'au moins filtre UV-A du type 4,4,-diarylbutadiène, sous agitation, une phase grasse d'une part et une phase aqueuse d'autre part, ledit mélange se faisant à une température supérieure à la température d'inversion de phase (TIP) du milieu, de manière à obtenir une émulsion de type eau-dans-huile. (ii) On ramène la température de l'émulsion ainsi obtenue à une température inférieure à ladite température d'inversion de phase, ce par quoi l'on obtient une émulsion ultrafine de type huile-dans-eau. (iii) On procède à l'introduction de nanopigments minéraux lors de la mise en oeuvre de l'étape (i) et/ou à l'issue de l'étape (ii).

L'une des difficultés pour la mise en oeuvre d'un procédé tel que ci-dessus réside dans le choix convenable du système émulsionnant qui doit être approprié au résultat recherché. Les systèmes convenables sont des émulsionnants de type non ioniques et choisis parmi les alcools gras polyoxyéthylénés et/ou polyoxypropylénés (i.e des composés obtenus par réaction entre un alcool gras aliphatique, comme l'alcool béhénique ou l'alcool cétylique, avec de l'oxyde d'éthylène ou de l'oxyde de propylène ou un mélange oxyde d'éthylène/oxyde de propylène) et les esters d'acides gras et de polyols, éventuellement polyoxyéthylénés et/ou polyoxypropylénés (i.e des composés obtenus par réaction d'un acide gras, comme l'acide stéarique ou l'acide oléique, avec un polyol, comme par exemple un alkylèneglycol ou du glycérol ou un polyglycérol, éventuellement en présence d'oxyde d'éthylène ou d'oxyde de propylène ou d'un mélange oxyde d'éthylène/oxyde de propylène), ou leurs mélanges. Par ailleurs, et de préférence, le système émulsionnant retenu présentera un HLB global (HLB = Hydrophilic-Lipophilic Balance, au sens de Griffin ; voir J. Soc. Cosm. Chem. 1954 (vol 5), pp 249-256 ; équilibre entre le caractère hydrophile et le caractère lipophile de l'agent tensioactif) allant de 9,5 à 11,5 environ, avantageusement proche de 10, de manière à permettre l'obtention d'une inversion de phase à une température inférieure à 90°C (TIP<90°C). La teneur en agent(s) émulsionnants est comprise entre 0,5 et 40% en poids et de préférence entre 2 et 10% en poids par rapport au poids total de l'émulsion.

Les compositions conformes à l'invention peuvent comporter en plus d'autres filtres UV organiques complémentaires actifs dans l'UVA et/ou l'UVB hydrosolubles ou liposolubles ou bien insolubles dans les solvants cosmétiques couramment utilisés. Les filtres organiques complémentaires sont notamment choisis parmi les anthranilates ; les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469, EP933376, EP507691, EP507692, EP790243, EP944624 ; les dérivés de la benzophénone ;

les dérivés de β,β-diphénylacrylate ; les dérivés de benzotriazole ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de benzoxazole tels que décrits dans les demandes de brevet EP0832642, EP1027883, EP1300137 et DE10162844 ; les dérivés de méthylène bis-(hydroxyphényl benzotriazole) tels que décrits dans s les demandes US 5,237,071, US 5,166,355, GB2303549, DE 197 26 184 et EP893119 ; les polymères filtres et silicones filtres tels que ceux décrits notamment dans la demande WO-93/04665 ; les dimères dérivés d'α-alkylstyrène tels que ceux décrits dans la demande de brevet DE19855649 et leurs mélanges.

Comme exemples de filtres organiques actifs dans l'UV-A et/ou l'UV-B, on peut citer désignés ci-dessus sous leur nom INCI :

Dérivés de l'acide para-aminobenzoique :
PABA,
Ethyl PABA,
Ethyl Dihydroxypropyl PABA,
Ethylhexyl Diméthyl PABA vendu notamment sous le nom « ESCALOL 507 » par ISP,
Glyceryl PABA,
PEG-25 PABA vendu sous le nom « UVINUL P25 » par BASF,

Dérivés salicyliques:
Homosalate vendu sous le nom « Eusolex HMS » par Rona/EM Industries,
Ethylhexyl Salicylate vendu sous le nom « NEO HELIOPAN OS » par Haarmann et REIMER,
Dipropyleneglycol Salicylate vendu sous le nom « DIPSAL » par SCHER,
TEA Salicylate, vendu sous le nom « NEO HELIOPAN TS » par Haarmann et REIMER,

Dérivés du dibenzoylméthane :
Butyl Methoxydibenzoylmethane vendu notamment sous le nom commercial « PARSOL 1789 » par HOFFMANN LAROCHE,
Isopropyl Dibenzoylmethane,

Dérivés cinnamiques :
Ethylhexyl Methoxycinnamate vendu notamment sous le nom commercial « PARSOL MCX » par HOFFMANN LAROCHE,
Isopropyl Methoxy cinnamate,
Isoamyl Methoxy cinnamate vendu sous le nom commercial « NEO HELIOPAN E 1000 » par HAARMANN et REIMER,
Cinoxate,
DEA Methoxycinnamate,
- Diisopropyl Methylcinnamate,
Glyceryl Ethylhexanoate Dimethoxycinnamate

Dérivés de β,β-diphénylacrylate :
Octocrylene vendu notamment sous le nom commercial « UVINUL N539 » par BASF,
Etocrylene, vendu notamment sous le nom commercial « UVINUL N35 » par BASF,

Dérivés de la benzophénone :
Benzophenone-1 vendu sous le nom commercial « UVINUL 400 » par BASF,
Benzophenone-2 vendu sous le nom commercial « UVINUL D50 » par BASF
Benzophenone-3 ou Oxybenzone, vendu sous le nom commercial « UVINUL M40 » par BASF,
Benzophenone-4 vendu sous le nom commercial « UVINUL MS40 » par BASF, Benzophenone-5
Benzophenone-6 vendu sous le nom commercial « Helisorb 11 » par Norquay
Benzophenone-8 vendu sous le nom commercial « Spectra-Sorb UV-24 » par American Cyanamid
Benzophenone-9 vendu sous le nom commercial« UVINUL DS-49» par BASF,
Benzophenone-12,
le 2-(4-diéthylamino-2-hydroxybenzoyl)-benzoate de n-hexyle

Dérivés du benzylidène camphre :
3-Benzylidene camphor fabriqué sous le nom « MEXORYL SD» par CHIMEX,
4-Methylbenzylidene camphor vendu sous le nom « EUSOLEX 6300 » par MERCK ,

Benzylidene Camphor Sulfonic Acid fabriqué sous le nom « MEXORYL SL» par CHIMEX, Camphor Benzalkonium Methosulfate fabriqué sous le nom « MEXORYL SO » par CHIMEX,
- Terephthalylidene Dicamphor Sulfonic Acid fabriqué sous le nom « MEXORYL SX » par CHIMEX,
Polyacrylamidomethyl Benzylidene Camphor fabriqué sous le nom « MESORYL SW » par CHIMEX,

Dérivés de benzimidazole :
Phenylbenzimidazole Sulfonic Acid vendu notamment sous le nom commercial « EUSOLEX 232 » par MERCK,
Disodium Phenyl Dibenzimidazole Tetra-sulfonate vendu sous le nom commercial commercial « NEO HELIOPAN AP » par Haarmann et REIMER,

Dérivés de triazine :
Anisotriazine vendu sous le nom commercial «TINOSORB S » par CIBA SPECIALTY CHEMICALS
Ethylhexyl triazone vendu notamment sous le nom commercial «UVINUL T150 » par BASF,
Diethylhexyl Butamido Triazone vendu sous le nom commercial « UVASORB HEB » par SIGMA 3V
la 2,4,6- tris-(4'amino-benzalmalonate de diisobutyle)-s-triazine.

Dérivés de benzotriazole :
Drometrizole Trisiloxane vendu sous le nom « Silatrizole » par RHODIA CHIMIE ,
Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

Dérivés anthraniliques :
Menthyl anthranilate vendu sous le nom commercial commercial « NEO HELIOPAN MA » par Haarmann et REIMER,

Dérivés d'imidazolines :
Ethylhexyl Dimethoxybenzylidene Dioxoimidazoline Propionate,

Dérivés de benzalmalonate :
Polyorganosiloxane à fonctions benzalmalonate tel que le polysilicone-15 vendu sous la dénomination commerciale « PARSOL SLX » par HOFFMANN LAROCHE

Dérivés de benzoxazole :
2,4-bis-[5-1 (diméthylpropyl)benzoxazol-2-yl-(4-phenyl)-imino]-6-(2-ethylhexyl)-imino-1,3,5-triazine vendu sous le nom Uvasorb K2A par Sigma 3V ;
et leurs mélanges.

Les filtres organiques plus particulièrement préférés sont choisis parmi les composés suivants :

Ethylhexyl Salicylate,
Ethylhexyl Methoxycinnamate
Octocrylene,
Butyl Methoxydibenzoylmethane
Phenylbenzimidazole Sulfonic Acid,
Benzophenone-3,
Benzophenone-4,
Benzophenone-5,
le 2-(4-diéthylamino-2-hydroxybenzoyl)-benzoate de n-hexyle
4-Methylbenzylidene camphor,
Terephthalylidene Dicamphor Sulfonic Acid,
Disodium Phenyl Dibenzimidazole Tetra-sulfonate,
la 2,4,6-tris-(4'-amino benzalmalonate de diisobutyle)-s-triazine Anisotriazine,
Ethylhexyl triazone,
Diethylhexyl Butamido Triazone,
Methylène bis-Benzotriazolyl Tetramethylbutylphénol
Drometrizole Trisiloxane
Polysilicone 15
2,4-bis-[5-1 (diméthylpropyl)benzoxazol-2-yl-(4-phenyl)-imino]-6-(2-ethylhexyl)-imino-1,3,5-triazine et leurs mélanges.

Les filtres complémentaires selon l'invention sont généralement présents dans les compositions selon l'invention à une teneur allant de 0,1 % à 30 % en poids et de préférence de 0,5 à 15 % , en poids, par rapport au poids total de la composition.

Les compositions selon l'invention peuvent également contenir des agents de bronzage et/ou de brunissage artificiels de la peau (agents autobronzants).

Les agents autobronzants sont généralement choisis parmi les composés mono ou polycarbonylés tels que par exemple l'isatine, l'alloxane, la ninhydrine, le glycéraldéhyde, l'aldéhyde mésotartrique, la glutaraldéhyde, l'érythrulose, les dérivés de pyrazolin-4,5-diones telles que décrites dans la demande de brevet FR 2 466 492 et WO 97/35842, la dihydroxyacétone (DHA), les dérivés de 4,4-dihydroxypyrazolin-5-ones telles que décrites dans la demande de brevet EP 903 342. On utilisera de préférence la DHA.

La DHA peut être utilisée sous forme libre et/ou encapsulée par exemple dans des vésicules lipidiques telle que des liposomes, notamment décrits dans la demande WO 97/25970.

Les agents autobronzants mono ou polycarbonylés sont généralement présents dans les compositions selon l'invention dans des proportions allant de 0,1 à 10% en poids par rapport au poids total de la composition, et de préférence de 0,2 à 8% en poids par rapport au poids total de la composition

Les compositions conformes à la présente invention peuvent comprendre en outre des adjuvants cosmétiques classiques notamment choisis parmi les solvants organiques, les épaississants ioniques ou non ioniques, les adoucissants, les humectants, les opacifiants, les stabilisants, les émollients, les silicones, les agents répulsifs contre les insectes, les parfums, les conservateurs, les tensioactifs, les charges, les pigments, les polymères, les propulseurs, les agents alcalinisants ou acidifiants ou tout autre ingrédient habituellement utilisé dans le domaine cosmétique et/ou dermatologique.

Bien entendu, l'homme de l'art veillera à choisir le ou les éventuels composés complémentaires cités ci-dessus et/ou leurs quantités de manière telle que les propriétés avantageuses attachées intrinsèquement aux émulsions ultrafines conformes à l'invention ne soient pas, ou substantiellement pas, altérées par la ou les adjonctions envisagées.

Parmi les solvants organiques, on peut citer les alcools et polyols inférieurs.

Parmi les épaississants on peut citer les polymères acryliques réticulés comme les Carbomers fournis par Novéon, les polymères réticulés acrylates/C10-30 alkylacrylates du type Pemulen fournis par Novéon ou le polyacrylate-3 vendu sous le nom Viscophobe DB 1000 par Amerchol) ; les polymères dérivés de l'acide acrylamido 2-méthylpropane sulfonique (Hostacerin AMPS fourni par Clariant, Sépigel 305 fourni par Seppic), les polymères neutres synthétiques tels que la poly N-vinylpyrrolidone, les polysaccharides comme les gommes de guar, de xanthane et les dérivés cellulosiques modifiés ou non comme la gomme de guar hydroxypropylée, la méthylhydroxyéthylcellulose et l'hydroxypropylméthylcellulose.

Les émulsions selon l'invention trouvent leur application dans un grand nombre de traitements, notamment cosmétiques, de la peau, des lèvres et des cheveux, y compris le cuir chevelu, notamment pour la protection et/ou le soin de la peau, des lèvres et/ou des cheveux, et/ou pour le maquillage de la peau et/ou des lèvres.

Un autre objet de la présente invention est constitué par l'utilisation des émulsions selon l'invention telles que ci-dessus définies pour la fabrication de compositions pour le traitement cosmétique de la peau, des lèvres et des cheveux, y compris le cuir chevelu, notamment pour la protection et/ou le soin de la peau, des lèvres et/ou des cheveux, et/ou pour le maquillage de la peau et/ou des lèvres.

Les compositions cosmétiques selon l'invention peuvent par exemple être utilisées comme produit de soin et/ou de protection solaire pour le visage et/ou le corps. Elles peuvent alors être conditionnées sous la forme de crèmes, de lait, de gels crèmes, ou bien encore de lotions fluides, en particulier de lotions fluides vaporisables (les compositions selon l'invention présentant en effet la propriété avantageuse complémentaire d'être aisément diluables à l'eau).

Les compositions selon l'invention sous forme de lotions fluides vaporisables conformes à l'invention sont appliquées sur la peau ou les cheveux sous forme de fines particules au moyen de dispositifs de pressurisation. Les dispositifs conformes à l'invention sont bien connus de l'homme de l'art et comprennent les pompes non-aérosols ou "atomiseurs", les récipients aérosols comprenant un propulseur ainsi que les pompes aérosols utilisant l'air comprimé comme propulseur. Ces derniers sont décrits dans les brevets US 4,077,441 et US 4,850,517.

Les compositions conditionnées en aérosol conformes à l'invention contiennent en général des agents propulseurs conventionnels tels que par exemple les composés hydrofluorés le dichlorodifluorométhane, le difluoroéthane, le diméthyléther, l'isobutane, le n-butane, le propane, le trichlorofluorométhane. Ils sont présents de préférence dans des quantités allant de 15 à 50% en poids par rapport au poids total de la composition.

Les exemples concrets, mais nullement limitatifs, illustrant l'invention, vont maintenant être donnés.

**EXEMPLES**

Le mode opératoire qui a été suivi pour préparer l'émulsion 1 était le suivant : les phases grasse (A) et aqueuse (B) ont été toutes deux préalablement portées à une température de l'ordre de 90°C ; puis on a introduit et dispersé la phase (C) contenant les nanopigments dans la phase grasse (A), et ceci sous agitation énergique de cette dernière au moyen d'une turbine type MORITZ (1000 t/mn) ; et enfin on a ajouté la phase aqueuse (A) dans la dispersion résultante, toujours sous agitation mécanique. Cette dernière étape d'émulsification a été conduite à 80°C, c'est à dire à une température supérieure à la température d'inversion de phase du système, laquelle est ici de 72°C (TIP).

L'émulsion 1 suivante a été préparée (% en poids ramenés à l'ensemble de la formulation) :

| Constituants | | Emulsion 1 (hors invention) |
|---|---|---|
| Phase A | Alcool stéarylique oxyéthyléné (12 OE) Emulgin B1 - Cognis) | 6,6 |
| | Stéarate de glycéryle (Tegin 90 - Goldschmidt) | 3,4 |
| | Huile de vaseline | 30 |
| | Polydiméthylsiloxane (Dow Corning 200 Fluid- Dow Corning)) | 0,5 |
| | Alcool cétéarylique/cétéarylglucoside (88/12) (Montanov 68- Seppic) | 2,0 |
| | Ethylhexyl methoxycinnamate (Uvinul MC 80- BASF) | 7,0 |
| | 1,1-dicarboxy(2'2'-dimethyl-propyl)-4,4-diphenylbutadiène | - |
| | Oxyde de titane $TiO_2$ nanopigmentaire (MT 100T - TAYCA) | 5,0 |
| Phase B | Glycérine | 5,0 |
| | Conservateurs | qs |
| | Eau déminéralisée | qsp 100 |
| Phase C | Oxyde de titane $TiO_2$ nanopigmentaire (MT 100T - TAYCA) | 5,0 |

L'émulsion 1 obtenue est stable dans le temps, présente une bonne rémanence à l'eau et un niveau d'efficacité dans l'UVA analogue à une composition du même type contenant l'acide benzène 1,4-di(3-méthylidène-10-camphosulfonique) et ses différents sels. Ses performances cosmétiques sont comparables à celles obtenues avec une émulsion huile/eau classique.

**Etude comparative de la rémanence à l'eau**

On réalise l'émulsion 2 huile/eau classique suivante (% en poids ramenés à l'ensemble de la formulation) :

| Constituants | | Emulsion 2 (hors invention) |
|---|---|---|
| Phase A | Mélange de monostéarate de glycéryle/stéarate de polyéthyleneglycol (100 OE) (Arlacel 165- Uniquema) | 1,0 |
| | Alcool cétylique (Lanette 16- Cognis) | 0,5 |
| | Acide gras d'origine végétale (Stéarine TP- Stéarinerie Dubois) | 1,5 |
| | Polydiméthylsiloxane (Dow Corning 200 Fluid- Dow Corning)) | 0,5 |
| | Alcool cétéarylique/cétéarylglucoside (88/12) (Montanov 68- Seppic) | 2,0 |
| | Benzoate d'alcools en $C_{12}$-$C_{15}$ (Finsolv TN - WITCO) | 5 |
| | Ethylhexyl methoxycinnamate (Uvinul MC 80- BASF) | 7 |
| | 1,1-dicarboxy(2'2'-dimethyl-propyl)-4,4-diphenylbutadiène | 5 |
| | Oxyde de titane $TiO_2$ nanopigmentaire (MT 100T - TAYCA) | 5 |

(suite)

| Constituants | | Emulsion 2 (hors invention) |
|---|---|---|
| Phase B | Glycérine | 5 |
| | Phosphate d'alcool hexadecylique, sel de potassium (Amphisol K-Roche Vitamins) | 1 |
| | Gomme de Xanthane (Keltrol T - CP Kelco) | 0,2 |
| | Acrylates/$C_{10}$-$C_{30}$ Alkylacrylates (Permulen TR 1 - Noveon) | 0,1 |
| | Triéthanolamine | qs |
| | Conservateurs | qs |
| | Eau déminéralisée | qsp 100 |

**Mode opératoire de fabrication :**

On pèse la phase grasse (A) et on chauffe au bain marie à 70 °C. On pèse la phase aqueuse (B) dans le bécher final et on chauffe au bain marie à 70°C. Sous agitation de type rotor (Moritz), on introduit la phase (A) dans la phase aqueuse (B). On laisse refroidir jusqu'à la température ambiant, on neutralise et on conditionne.

On réalise ensuite l'émulsion 3 huile/eau suivante contenant le filtre UV-A : Acide benzène 1,4-di(3-méthylidène-10-camphosulfonique) selon la technique d'inversion de phase dans les mêmes conditions que l'exemple 1 (% en poids ramenés à l'ensemble de la formulation) :

| Constituants | | Emulsion 3 (hors invention) |
|---|---|---|
| Phase A | Alcool stéarylique oxyéthyléné (12 OE) Emulgin B1 - Cognis) | 6,6 |
| | Stéarate de glycéryle (Tegin 90 - Goldschmidt) | 3,4 |
| | Huile de vaseline | 30 |
| | Polydiméthylsiloxane (Dow Corning 200 Fluid- Dow Corning)) | 0,5 |
| | Alcool cétéarylique/cétéarylglucoside (88/12) (Montanov 68- Seppic) | 2,0 |
| | Ethylhexyl methoxycinnamate (Uvinul MC 80- BASF) | 7,0 |
| | 1,1-dicarboxy(2'2'-dimethyl-propyl)-4,4-diphenylbutadiène | - |
| | Oxyde de titane $TiO_2$ nanopigmentaire (MT100T-TAYCA) | 5,0 |
| Phase B | Glycérine | 5,0 |
| | Acide benzène 1,4-di(3-méthylidène-10-camphosulfonique) (Mexoryl SX - Chimex) | 5,0 |
| | Conservateurs | qs |
| | Eau déminéralisée | qsp 100 |
| Phase C | Oxyde de titane $TiO_2$ nanopigmentaire (MT 100T - TAYCA) | 5,0 |

On a mesuré et comparé la rémanence à l'eau du facteur de protection solaire (SPF) des émulsions 1, 2 et 3 telles que définies précédemment selon le protocole suivant :

| | |
|---|---|
| Support d'étalement : | Plaques du type Transpore® monté sur un cadre de diapositive |
| Quantité déposée : | 30 mg |
| Application | au doigt nu sur le recto et le verso de la plaque |
| Quantité par application : | 19 mg ± 0,5 (soit 0,75 mg /$cm^2$) |
| Nombre d'échantillons : | 4 mesures par application et 5 applications par formule |
| Bain : | Immersion et agitation des échantillons dans un bain d'eau à 30°C pendant 10 mn |

Détermination des SPF avant et après bain

| | |
|---|---|
| Appareil de mesure | Spectroradiomètre SPF-290 (Optometrics) Enregistrement des facteurs de protection solaire monochromatique tous les 5 nm entre 290 et 400 nm |
| Mode de calcul des SPF | Méthode décrite par B. L. Diffey et al dans J. Soc. Cosmet. 40-127-133 (1989) |
| Spectre de source : | Soleil de Diffey |
| Spectre d'action | Erythème CIE 1987 |

On calcule ensuite le pourcentage d'efficacité résiduelle après bain selon l'équation suivante :

**% efficacité résiduelle = 100X (SPF moyen après bain – 1) / SPF moyen après bain – 1**

Les résultats obtenus sur les émulsions 1, 2 et 3 sont les suivantes :

| Composition | % efficacité résiduelle après bain |
|---|---|
| Emulsion 1 (hors invention) | 68% |
| Emulsion 2 (hors invention) | 51% |
| Emulsion 3 (hors invention) | 27% |

On constate que l'émulsion 1 obtenue selon la technique d'inversion de phase comme filtre UV-A un composé 4,4-diarylbutadiène présente une rémanence à l'eau 2,5 fois plus importante que l'émulsion 3 obtenue selon la technique d'inversion de phase contenant des nanopigments de $TiO_2$ et comme filtre UV-A d'efficacité équivalente : l'acide benzène 1,4-di(3-méthylidène-10-camphosulfonique).

On constate que l'émulsion 1 obtenue selon la technique d'inversion de phase contenant des nanopigments de $TiO_2$ et comme filtre UV-A un composé 4,4-diarylbutadiène présente meilleure rémanence à l'eau par rapport à l'émulsion 2 du type huile /eau classique contenant le même système filtrant.

## Revendications

1. Emulsion huile-dans-eau susceptible d'être obtenue selon la technique d'inversion de phase dans laquelle la taille moyenne des globules qui constituent la phase huileuse de ladite émulsion est comprise entre 100 nm et 1000 nm et comprenant des nanopigments minéraux à base d'oxydes métalliques et au moins un filtre UV organique, **caractérisée par le fait qu'**elle contient au moins un filtre UV-A organique du type 4,4-diarylbutadiène.

2. Emulsion selon la revendication 1, **caractérisée en ce que** ladite taille moyenne des globules huileux est comprise entre 100 et 500 nm.

3. Emulsion selon l'une quelconque des revendications 1 ou 2, **caractérisée en ce qu**'au moins 90 % en nombres desdits globules présentent une taille comprise entre 100 et 1000 nm, de préférence entre 100 et 500 nm.

4. Emulsion selon l'une quelconque des revendications 1 à 3, **caractérisées en ce que** lesdits nanopigments sont choisis parmi les oxydes de titane, de zinc, de fer, de zirconium, de cérium et leurs mélanges, enrobés ou non.

5. Emulsion selon la revendication 4, **caractérisée en ce que** les nanopigments sont à base d'oxyde de titane, enrobé ou non enrobé.

6. Emulsion selon la revendication 5, **caractérisée en ce que** l'oxyde de titane est sous forme rutile, anatase ou amorphe.

7. Emulsion selon l'une quelconque des revendications 1 à 6, **caractérisée en ce que** la teneur en poids des nanopigments représente de 0,5 à 40 %, de préférence de 1 à 30 %, du poids total de la composition.

**8.** Emulsion selon l'une quelconque des revendications 1 à 7, où le filtre UV-A du type 4,4-diarylbutadiène répond à la formule (I) suivante :

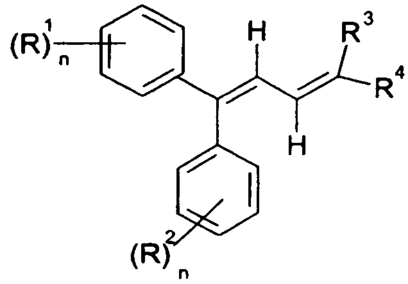

(I)

dans laquelle le système diène est de configuration Z,Z ; Z,E ; E,Z ou E,E ou des mélanges desdites configurations et où :

- $R^1$ et $R^2$ identiques ou différents, désignent hydrogène, un radical alkyle en C1-C20,; un radical alcényle en $C_2$- $C_{10}$ ; un radical alcoxy en $C_1$-$C_{12}$ ; un radical cycloalkyle en $C_3$-$C_{10}$; un radical cycloalcényle en $C_3$-$C_{10}$ ; un radical alcoxycarbonyle en $C_1$-$C_{20}$ ; un radical monoalkylamino en $C_1$-$C_{12}$ ; un radical dialkylamino en $C_1$-$C_{12}$ ; un aryle ; un hétéroaryle ou un substituant hydrosolubilisant choisi parmi un reste carboxylate, sulfonate ou un reste ammonium ;
- $R^3$ désigne un groupe $COOR^5$; $COR^5$; $CONR^5R^6$; CN ; O=S(-$R^5$)=O; O=S(-$OR^5$)=O; $R^7$O-P-(-$OR^8$)=O; un radical alkyle en $C_1$-$C_{20}$ ; un radical alcényle en $C_2$- $C_{10}$; un radical cycloalkyle en $C_3$-$C_{10}$; un radical bicycloalkyle en $C_7$-$C_{10}$; un radical cycloalcényle en $C_3$-$C_{10}$; un radical bicycloalcényle en $C_7$-$C_{10}$; un aryle en $C_6$-$C_{18}$ éventuellement substitué ; un hétéroaryle en $C_3$-$C_7$ éventuellement substitué;
- $R^4$ désigne un groupe $COOR^6$; $COR^6$; $CONR^5R^6$; CN ; O=S(-$R^6$)=O ; O=S(-$OR^6$)=O ; $R^7$O-P-(-$OR^8$)=O; un radical alkyle en $C_1$-$C_{20}$ ; un radical alcényle en $C_2$- $C_{10}$ ; un radical cycloalkyle en $C_3$-$C_{10}$ ; un radical bicycloalkyle en $C_7$-$C_{10}$ ; un radical cycloalcényle en $C_3$-$C_{10}$; un radical bicycloalcényle en $C_7$-$C_{10}$; un aryle en $C_6$-$C_{18}$ éventuellement substitué ; un hétéroaryle en $C_3$-$C_7$ éventuellement substitué;
- les radicaux $R^5$ à $R^8$, identiques ou différents, désignent hydrogène ; un radical alkyle en $C_1$-$C_{20}$ ; un radical alcényle en $C_2$-$C_{10}$; un radical cycloalkyle en $C_3$-$C_{10}$; un radical bicycloalkyle en $C_7$-$C_{10}$; un radical bicycloalcényle en $C_3$-$C_{10}$ ; un radical cycloalcényle en $C_7$-$C_{10}$ ; un aryle éventuellement substitué ; un hétéroaryle éventuellement substitué ;
- n varie de 1 à3;

les radicaux $R^3$ à $R^8$ peuvent former entre eux avec les atomes de carbone auxquels ils sont liés, un noyau en $C_5$-$C_6$ pouvant être condensé.

**9.** Emulsion selon la revendication 8, où le composé de formule (I) est chois parmi ceux pour lesquels

- n = 1 ou 2 ;
- $R^1$ et $R^2$, identiques ou différents, désignent hydrogène, un radical alkyle en $C_1$-$C_{20}$; un radical alcoxy en $C_1$-$C_{12}$ ; un radical monoalkylamino en $C_1$-$C_{12}$ ; un radical dialkylamino en $C_1$-$C_{12}$ ; un substituant hydrosolubilisant choisi parmi un groupe carboxylate, un groupe sulfonate ou un reste ammonium ;
- $R^3$ désigne un groupe $COOR^5$; $COR^5$; $CONR^5R^6$ ; un radical alkyle en $C_1$-$C_{20}$ ; un radical cycloalkyle en $C_3$-$C_{10}$ ; un radical cycloalcényle en $C_3$-$C_{10}$ un radical bicycloalkyle en $C_7$-$C_{10}$ ; phényle, naphtyle ou thienyle éventuellement substitué ;
- $R^4$ désigne un groupe $COOR^6$ ; $COR^6$; $CONR^5R^6$ ; un radical alkyle en $C_1$-$C_{20}$ ; un radical cycloalkyle en $C_3$-$C_6$ ; un radical cycloalcényle en $C_3$-$C_{10}$ ; un radical bicycloalkyle en $C_7$-$C_{10}$ ; phényle, naphtyle ou thienyle éventuellement substitué ;
- les radicaux R et $R^6$, identiques ou différents, désignent hydrogène ; un radical alkyle en $C_1$-$C_{12}$ ; un radical cycloalkyle en $C_3$-$C_{10}$; un radical cycloalcényle en $C_3$-$C_{10}$ ; un radical bicycloalkyle en $C_7$-$C_{10}$; un radical bicycloalcényle en $C_3$-$C_{10}$ ; phényle ou naphtyle éventuellement substitué.

**10.** Emulsion selon la revendication 9, où le composé de formule (I) est chois parmi ceux pour lesquels

- $R^1$ et $R^2$, identiques ou différents, désignent hydrogène, un radical alkyle en $C_1$-$C_{20}$ ; un radical alcoxy en $C_1$-$C_{20}$; un substituant hydrosolubilisant choisi parmi un groupe carboxylate, un groupe sulfonate ou un reste ammonium ;
- $R^3$ désigne un groupe $COOR^5$; $COR^5$; $CONR^5R^6$ ;
- $R^4$ désigne un groupe $COOR^6$ ; $COR^6$ ; $CONR^5R^6$ ;
- les radicaux R et $R^6$, identiques ou différents, désignent hydrogène ; un radical alkyle en $C_1$-$C_{12}$ ; un radical cycloalkyle en $C_3$-$C_6$ ; un radical cycloalcényle en $C_3$-$C_{10}$ ; un radical bicycloalkyle en $C_7$-$C_{10}$; un radical bicycloalcényle en $C_3$-$C_{10}$ ; phényle ou naphtyle éventuellement substitué.

**11.** Emulsion selon la revendication 10, où le composé de formule (I) est choisi parmi ceux de formule (I') suivante :

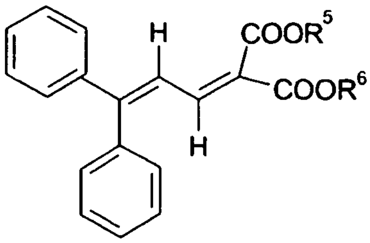

(I')

où les radicaux $R^5$ et $R^6$, identiques ou différents, désignent hydrogène ; un radical alkyle en $C_1$-$C_{20}$; un radical cycloalkyle en $C_3$-$C_6$ ; un radical cycloalcényle en $C_3$-$C_{10}$.

**12.** Emulsion selon la revendication 11, où le composé de formule (I') est le dérivé 1,1-dicarboxy(2'2'-diméthyl-propyl)-4,4-diphénylbutadiène de structure :

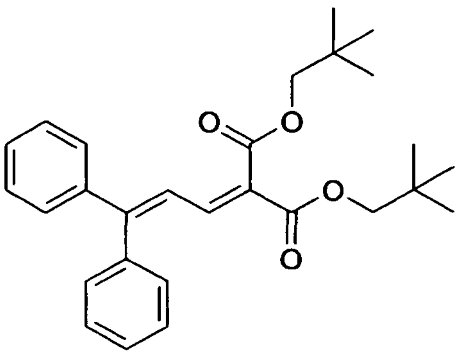

(composé f)

**13.** Emulsion selon l'une quelconque des revendications 1 à 7, où le filtre UV-A du type 4,4-diarylbutadiène répond à la formule (II) suivante :

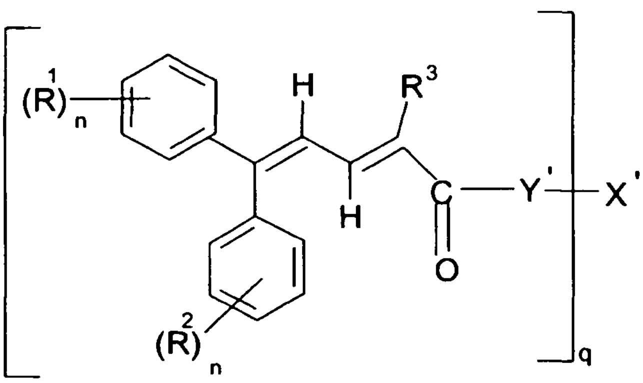

(II)

dans laquelle le système diène est de configuration Z,Z ; Z,E ; E,Z ou E,E ou des mélanges desdites configurations et où :

- $R^1$, $R^2$, $R^3$ et n ont les mêmes significations indiquées dans la formule (I) telle que définie dans la revendication 8;
- Y' désigne un groupe -O- ou -$NR^9$-
- $R^9$ désigne hydrogène ; un radical alkyle en $C_1$-$C_{20}$, linéaire ou ramifié ; un radical alcényle en $C_2$- $C_{10}$ ; un radical cycloalkyle en $C_3$-$C_{10}$ ; un radical bicycloalkyle en $C_7$-$C_{10}$ : un radical cycloalcényle en $C_3$-$C_{10}$ ; ; un radical bicycloalcényle en $C_7$-$C_{10}$ ; un aryle ; un hétéroaryle;
- X' désigne un reste de polyol en $C_2$-$C_{20}$ linéaire ou ramifié, aliphatique ou cycloaliphatique comprenant de 2 à10 groupes hydroxy et de valence q ; la chaîne carbonée dudit reste pouvant être interrompue par un ou plusieurs atomes de souffre ou d'oxygène ; un ou plusieurs groupes imines ; un ou plusieurs alkylimino en $C_1$-$C_4$ ;
- q varie de 2 à 10.

**14.** Emulsion selon la revendication 13, où le composé de formule (II) est choisi parmi ceux pour lesquels :

- $R^1$ et $R^2$, identiques ou différents, désignent hydrogène, un radical alkyle en $C_1$-$C_{12}$ ; un radical alcoxy en $C_1$-$C_8$ ; un substituant hydrosolubilisant choisi parmi un groupe carboxylate, un groupe sulfonate ou un reste ammonium ;
- $R^3$ désigne un groupe $COOR^5$; $CONR^5R^6$; CN ; un radical cycloalkyle en $C_3$-$C_{10}$; un radical bicycloalkyle en $C_7$-$C_{10}$ ;
- $R^5$ et $R^6$ , identiques ou différents, désignent un radical alkyle en $C_1$-$C_{20}$, linéaire ou ramifié ; un radical cycloalkyle en $C_3$-$C_{10}$ ; un radical bicycloalkyle en $C_7$-$C_{10}$; naphtyle ou phényle éventuellement substitué ;
- X' désigne un reste de polyol en $C_2$-$C_{20}$ comprenant de 2 à 6 groupes hydroxy et plus particulièrement de 2 à 4.

**15.** Emulsion selon la revendication 14, où le composé de formule (II) est choisi parmi ceux pour lesquels X' désigne un reste d'éthanol ou de pentaerythritol.

**16.** Emulsion selon la revendication 15, où le composé de formule (II) est choisi parmi les composés suivants :

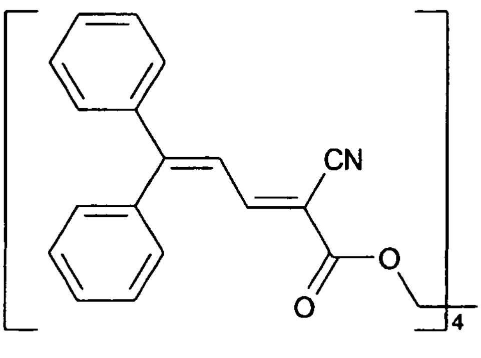

(composé g)

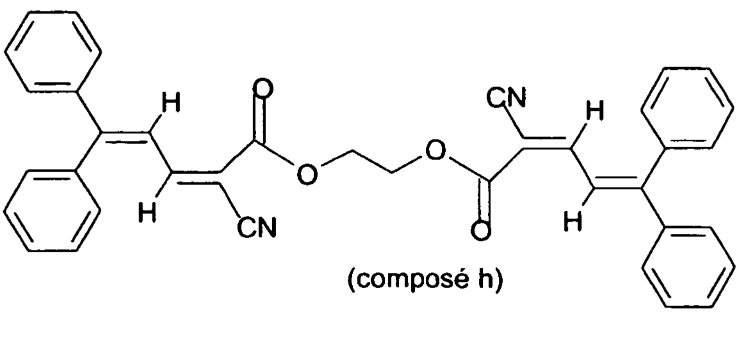

(composé h)

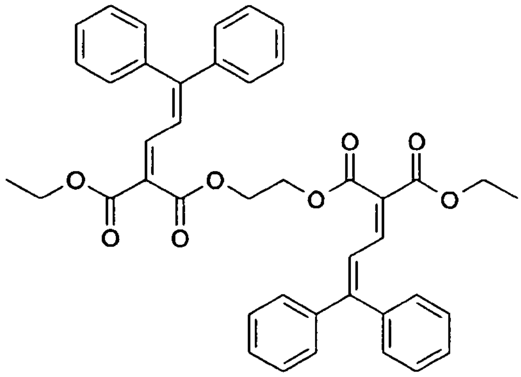

(composé i)

**17.** Emulsion selon l'une quelconque des revendications 1 à 16, **caractérisée en ce que** le ou les composés 4,4-diarylbutadiène sont présents dans des proportions allant de 0,1 % à 20% en poids, plus préférentiellement de 1 à 10% en poids par rapport au poids total de l'émulsion.

**18.** Emulsion selon l'une quelconque des revendications 1 à 17, **caractérisée en ce qu'**elle contient en outre au moins un filtre solaire organique complémentaire actif dans l'UV-A et/ou l'UV-B, hydrosolubles, liposolubles ou bien insolubles dans les solvants cosmétiques couramment utilisés.

**19.** Emulsion selon la revendication 18, ou les filtres organiques complémentaires sont choisis parmi les anthranilates; les dérivés cinnamiques; les dérivés de dibenzoylméthane ; les dérivés salicyliques, les dérivés du camphre ; les dérivés de triazine ; les dérivés de la benzophénone ; les dérivés de β,β-diphénylacrylate; les dérivés de benzotriazole ; les dérivés de benzalmalonate ; les dérivés de benzimidazole ; les imidazolines; les dérivés bis-benzoazolyle; les dérivés de l'acide p-aminobenzoïque (PABA) ; les dérivés de benzoxazole ; les dérivés de méthylène bis-(hydroxyphényl benzotriazole) ; les polymères filtres et silicones filtres ; les dimères dérivés d'α-alkylstyrène et leurs mélanges.

**20.** Emulsion selon la revendication 19, ou les filtres organiques complémentaires sont choisis parmi
Ethylhexyl Salicylate,
Ethylhexyl Methoxycinnamate
Octocrylene,
Butyl Methoxydibenzoylmethane
Phenylbenzimidazole Sulfonic Acid,
Benzophenone-3,
Benzophenone-4,
Benzophenone-5,
le 2-(4-diéthylamino-2-hydroxybenzoyl)-benzoate de n-hexyle
4-Methylbenzylidene camphor,
Terephthalylidene Dicamphor Sulfonic Acid,
Disodium Phenyl Dibenzimidazole Tetra-sulfonate,
la 2,4,6-tris-(4'-amino benzalmalonate de diisobutyle)-s-triazine
Anisotriazine,
Ethylhexyl triazone,
Diethylhexyl Butamido Triazone,
Methylène bis-Benzotriazolyl Tetramethylbutylphénol
Drometrizole Trisiloxane
Polysilicone-15
2,4-bis-[5-1(diméthylpropyl)benzoxazol-2-yl-(4-phenyl)-imino]-6-(2-ethylhexyl)-imino-1,3,5-triazine et leurs mélanges.

**21.** Emulsion selon l'une quelconque des revendications 1 à 20, **caractérisée en ce qu'**elle contient en outre au moins un agent de bronzage et/ou de brunissage artificiel de la peau.

**22.** Emulsion selon l'une quelconque des revendications 1 à 21, **caractérisée en ce qu'**elle contient en outre au moins un adjuvant cosmétique choisi parmi les solvants organiques, les épaississants ioniques ou non ioniques, les adoucissants, les humectants, les opacifiants, les stabilisants, les émollients, les silicones, les agents répulsifs contre les insectes, les parfums, les conservateurs, les tensioactifs, les charges, les pigments, les polymères, les propulseurs, les agents alcalinisants ou acidifiants ou tout autre ingrédient habituellement utilisé dans le domaine cosmétique et/ou dermatologique.

**23.** Emulsion selon l'une quelconque des revendications 1 à 22, **caractérisées en ce qu**'elles contient un système émulsionnant.

**24.** Emulsion selon la revendication 23 où le système émulsionnant comprend un ou plusieurs agents émulsionnants de type non ionique et choisis parmi les alcools gras polyoxyéthylénés eUou polyoxypropylénés, et les esters d'acides gras et de polyols, éventuellement polyoxyéthylénés et/ou polyoxypropylénés.

**25.** Emulsion selon la revendication 24 où le système émulsionnant présente un HLB global allant de 9,5 à 11,5 environ et de préférence proche de 10.

**26.** Emulsion selon la revendication 25, **caractérisée en ce que** la teneur en agent(s) émulsionnant(s) est comprise entre 0,5 et 40 % en poids, de préférence entre 2 et 10 % en poids, par rapport au poids total de l'émulsion.

**27.** Emulsion selon l'une quelconque des revendications 1 à 26, **caractérisée en ce que** la teneur en poids de la phase aqueuse représente de 50 à 95 %, de préférence de 70 à 90 %, du poids total de l'émulsion.

**28.** Emulsion selon l'une quelconque des revendications 1 à 27, **caractérisée en ce que** la teneur en poids de la phase huileuse représente de 5 à 50 %, de préférence de 10 à 30 %, du poids total de la composition.

**29.** Procédé de préparation d'une émulsion huile-dans-eau telle que définie à l'une quelconque des revendications 1 à 28, **caractérisé par le fait qu**'il comprend les étapes suivantes :

(i) on mélange, en présence d'un système émulsionnant convenable et d'au moins filtre UV-A du type 4,4,-diarylbutadiène, sous agitation, une phase grasse d'une part et une phase aqueuse d'autre part, ledit mélange se faisant à une température supérieure à la température d'inversion de phase (TIP) du milieu, de manière à obtenir une émulsion de type eau-dans-huile,
(ii) on ramène la température de l'émulsion ainsi obtenue à une température inférieure à ladite température d'inversion de phase, ce par quoi l'on obtient une émulsion ultrafine de type huile-dans-eau,
(iii) on procède à l'introduction de nanopigments minéraux lors de la mise en oeuvre de l'étape (i) et/ou à l'issue de l'étape (ii).

**30.** Utilisation d'une émulsion définie à l'une quelconque des revendications 1 à 28 pour la fabrication d'une composition pour le traitement cosmétique de la peau, des lèvres et des cheveux et du cuir chevelu.

**31.** Utilisation d'une émulsion définie à l'une quelconque des revendications 1 à 28, pour la fabrication d'un produit pour la protection et/ou le soin de la peau, des lèvres et/ou des cheveux et/ou pour le maquillage de la peau et/ou des lèvres.

**32.** Utilisation d'un filtre UV-A du type 4,4-diarylbutadiène tel que défini dans les revendications 1 à 15 dans une émulsion huile-dans-eau susceptible d'être obtenue selon la technique d'inversion de phase dans laquelle la taille moyenne des globules qui constituent la phase huileuse de ladite émulsion est comprise entre 100 nm et 1000 nm et comprenant des nanopigments minéraux à base d'oxydes métalliques et au moins un filtre UV organique, dans le but d'améliorer la rémanence à l'eau du facteur de protection solaire

## Claims

**1.** Oil-in-water emulsion capable of being obtained according to the phase inversion technique, in which the average size of the globules which constitute the oily phase of the said emulsion is between 100 nm and 1 000 nm and comprising inorganic nanopigments based on metal oxides and at least one organic UV-screening agent, **characterized in that** it contains at least one organic UV-A-screening agent of the 4,4-diarylbutadiene type.

**2.** Emulsion according to Claim 1, **characterized in that** the said average size of the oily globules is between 100 and 500 nm.

**3.** Emulsion according to either of Claims 1 and 2, **characterized in that** at least 90% in numerical terms of the said globules have a size of between 100 and 1 000 nm, preferably between 100 and 500 nm.

**4.** Emulsion according to any one of Claims 1 to 3, **characterized in that** the said nanopigments are chosen from titanium, zinc, iron, zirconium and cerium oxides, and mixtures thereof, coated or uncoated.

**5.** Emulsion according to Claim 4, **characterized in that** the nanopigments are based on titanium oxide, coated or uncoated.

**6.** Emulsion according to Claim 5, **characterized in that** the titanium oxide is in rutile, anatase or amorphous form.

**7.** Emulsion according to any one of Claims 1 to 6, **characterized in that** the content by weight of the nanopigments represents from 0.5 to 40%, preferably from 1 to 30%, of the total weight of the composition.

**8.** Emulsion according to any one of Claims 1 to 7, where the UV-A-screening agent of the 4,4-diarylbutadiene type corresponds to the following formula (I):

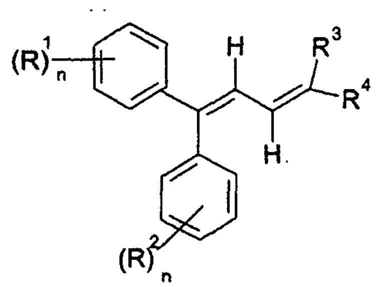

(I)

in which the diene system is of the Z,Z; Z,E; E,Z or E,E configuration or mixtures of the said configurations, and where:

- $R^1$ and $R^2$, which are identical or different, denote hydrogen, a $C_1$-$C_{20}$ alkyl radical; a $C_2$-$C_{10}$ alkenyl radical; a $C_1$-$C_{12}$ alkoxy radical; a $C_3$-$C_{10}$ cycloalkyl radical; a $C_3$-$C_{10}$ cycloalkenyl radical; a $C_1$-$C_{20}$ alkoxycarbonyl radical; a $C_1$-$C_{12}$ monoalkylamino radical; a $C_1$-$C_{12}$ dialkylamino radical; an aryl; a heteroaryl or a water-solubilizing substituent chosen from a carboxylate residue, a sulphonate residue or an ammonium residue;
- $R^3$ denotes a group $COOR^5$; $COR^5$; $CONR^5R^6$; CN; $O=S(-R^5)=O$; $O=S(-OR^5)=O$; $R^7O-P-(-OR^8)=O$; a $C_1$-$C_{20}$ alkyl radical; a $C_2$-$C_{10}$ alkenyl radical; a $C_3$-$C_{10}$ cycloalkyl radical; a $C_7$-$C_{10}$ bicycloalkyl radical; a $C_3$-$C_{10}$ cycloalkenyl radical; a $C_7$-$C_{10}$ bicycloalkenyl radical; an optionally substituted $C_6$-$C_{18}$ aryl; an optionally substituted $C_3$-$C_7$ heteroaryl;
- $R^4$ denotes a group $COOR^6$; $COR^6$; $CONR^5R^6$; CN; $O=S(-R^6)=O$; $O=S(-OR^6)=O$; $R^7O-P-(-OR^8)=O$; a $C_1$-$C_{20}$ alkyl radical; a $C_2$-$C_{10}$ alkenyl radical; a $C_3$-$C_{10}$ cycloalkyl radical; a $C_7$-$C_{10}$ bicycloalkyl radical; a $C_3$-$C_{10}$ cycloalkenyl radical; a $C_7$-$C_{10}$ bicycloalkenyl radical; an optionally substituted $C_6$-$C_{18}$ aryl; an optionally substituted $C_3$-$C_7$ heteroaryl;
- the radicals $R^5$ to $R^8$, which are identical or different, denote hydrogen; a $C_1$-$C_{20}$ alkyl radical; a $C_2$-$C_{10}$ alkenyl radical; a $C_3$-$C_{10}$ cycloalkyl radical; a $C_7$-$C_{10}$ bicycloalkyl radical; a $C_3$-$C_{10}$ bicycloalkenyl radical; a $C_7$-$C_{10}$ cycloalkenyl radical; an optionally substituted aryl; an optionally substituted heteroaryl;
- n varies from 1 to 3;

the radicals $R^3$ to $R^8$ can form between them, with the carbon atoms to which they are attached, a $C_5$-$C_6$ ring which may be fused.

**9.** Emulsion according to Claim 8, where the compound of formula (I) is chosen from those for which

- n = 1 or 2;
- $R^1$ and $R^2$, which are identical or different, denote hydrogen, a $C_1$-$C_{20}$ alkyl radical; a $C_1$-$C_{12}$ alkoxy radical; a $C_1$-$C_{12}$ monoalkylamino radical; a $C_1$-$C_{12}$ dialkylamino radical; a water-solubilizing substituent chosen from a carboxylate group, a sulphonate group or an ammonium residue;
- $R^3$ denotes a group $COOR^5$; $COR^5$; $CONR^5R^6$; a $C_1$-$C_{20}$ alkyl radical; a $C_3$-$C_{10}$ cycloalkyl radical; a $C_3$-$C_{10}$ cycloalkenyl radical; a $C_7$-$C_{10}$ bicycloalkyl radical; optionally substituted phenyl, naphthyl or thienyl;

- $R^4$ denotes a group $COOR^6$; $COR^6$; $CONR^5R^6$; a $C_1$-$C_{20}$ alkyl radical; a $C_3$-$C_6$ cycloalkyl radical; a $C_3$-$C_{10}$ cycloalkenyl radical; a $C_7$-$C_{10}$ bicycloalkyl radical; optionally substituted phenyl, naphthyl or thienyl;
- the radicals $R^5$ and $R^6$, which are identical or different, denote hydrogen; a $C_1$-$C_{12}$ alkyl radical; a $C_3$-$C_{10}$ cycloalkyl radical; a $C_3$-$C_{10}$ cycloalkenyl radical; a $C_7$-$C_{10}$ bicycloalkyl radical; a $C_3$-$C_{10}$ bicycloalkenyl radical; optionally substituted phenyl or naphthyl.

**10.** Emulsion according to Claim 9, where the compound of formula (I) is chosen from those for which

- $R^1$ and $R^2$, which are identical or different, denote hydrogen, a $C_1$-$C_{20}$ alkyl radical; a $C_1$-$C_{20}$ alkoxy radical; a water-solubilizing substituent chosen from a carboxylate group, a sulphonate group or an ammonium residue;
- $R^3$ denotes a group $COOR^5$; $COR^5$; $CONR^5R^6$;
- $R^4$ denotes a group $COOR^6$; $COR^6$; $CONR^5R^6$;
- the radicals $R^5$ and $R^6$, which are identical or different, denote hydrogen; a $C_1$-$C_{12}$ alkyl radical; a $C_3$-$C_6$ cycloalkyl radical; a $C_3$-$C_{10}$ cycloalkenyl radical; a $C_7$-$C_{10}$ bicycloalkyl radical; a $C_3$-$C_{10}$ bicycloalkenyl radical; optionally substituted phenyl or naphthyl.

**11.** Emulsion according to Claim 10, where the compound of formula (I) is chosen from those of the following formula (I'):

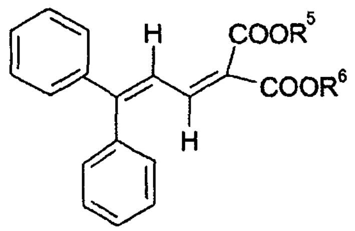

(I')

where the radicals $R^5$ and $R^6$, which are identical or different, denote hydrogen; a $C_1$-$C_{20}$ alkyl radical; a $C_3$-$C_6$ cycloalkyl radical; a $C_3$-$C_{10}$ cycloalkenyl radical.

**12.** Emulsion according to Claim 11, where the compound of formula (I') is the 1,1-dicarboxy-(2'2'-dimethylpropyl)-4,4-diphenylbutadiene derivative having the structure:

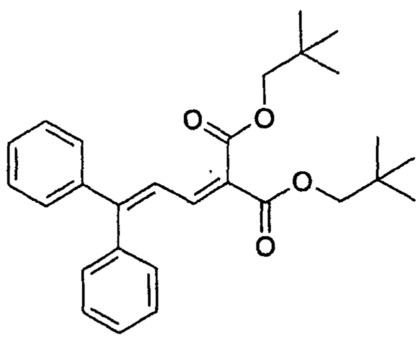

(compound f)

**13.** Emulsion according to any one of Claims 1 to 7, where the UV-A-screening agent of the 4,4-diarylbutadiene type corresponds to the following formula (II):

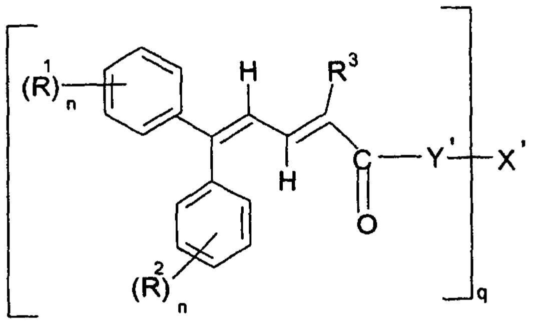

(II)

in which the diene system is of the Z, Z; Z,E; E,Z or E,E configuration or mixtures of the said configurations and where:

- $R^1$, $R^2$, $R^3$ and n have the meanings indicated in the formula (I) as defined in Claim 8;
- Y' denotes a group -O- or $-NR^9-$
- $R^9$ denotes hydrogen; a linear or branched $C_1$-$C_{20}$ alkyl radical; a $C_2$-$C_{10}$ alkenyl radical; a $C_3$-$C_{10}$ cycloalkyl radical; a $C_7$-$C_{10}$ bicycloalkyl radical; a $C_3$-$C_{10}$ cycloalkenyl radical; a $C_7$-$C_{10}$ bicycloalkenyl radical; an aryl; a heteroaryl;
- X' denotes a residue of a linear or branched, aliphatic or cycloaliphatic $C_2$-$C_{20}$ polyol comprising from 2 to 10 hydroxyl groups and having the valency q; it being possible for the carbon chain of the said residue to be interrupted by one or more sulphur or oxygen atoms; one or more imine groups; one or more $C_1$-$C_4$ alkylimino groups;
- q ranges from 2 to 10.

**14.** Emulsion according to Claim 13, where the compound of formula (II) is chosen from those for which:

- $R^1$ and $R^2$, which are identical or different, denote hydrogen, a $C_1$-$C_{12}$ alkyl radical; a $C_1$-$C_8$ alkoxy radical; a water-solubilizing substituent chosen from a carboxylate group, a sulphonate group or an ammonium residue;
- $R^3$ denotes a group $COOR^5$; $CONR^5R^6$; CN; a $C_3$-$C_{10}$ cycloalkyl radical; a $C_7$-$C_{10}$ bicycloalkyl radical;
- $R^5$ and $R^6$, which are identical or different, denote a linear or branched $C_1$-$C_{20}$ alkyl radical; a $C_3$-$C_{10}$ cycloalkyl radical; a $C_7$-$C_{10}$ bicycloalkyl radical; optionally substituted naphthyl or phenyl;
- X' denotes a $C_2$-$C_{20}$ polyol residue comprising from 2 to 6 hydroxyl groups and more particularly from 2 to 4.

**15.** Emulsion according to Claim 14, where the compound of formula (II) is chosen from those for which X' denotes an ethanol or pentaerythrol residue.

**16.** Emulsion according to Claim 15, where the compound of formula (II) is chosen from the following compounds:

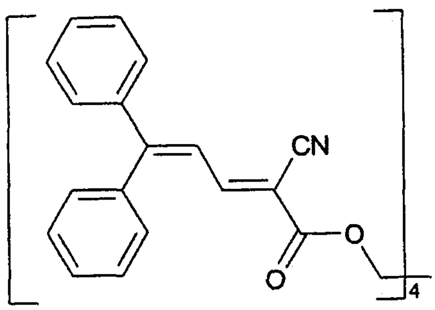

(compound g)

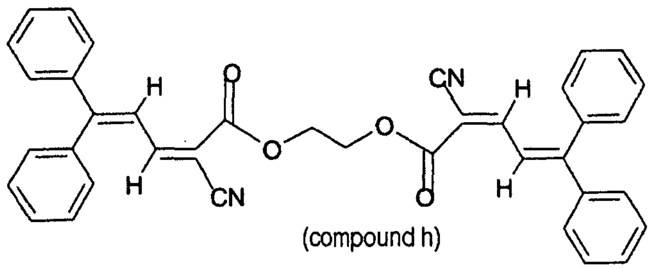

(compound h)

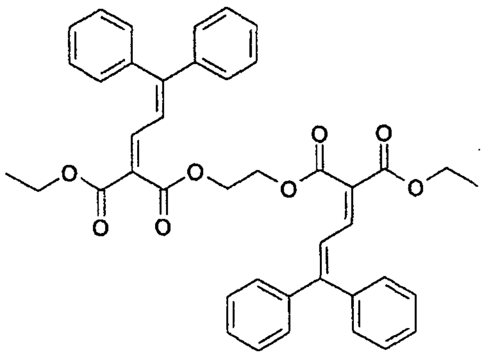

(compound i)

17. Emulsion according to any one of Claims 1 to 16, **characterized in that** the 4,4-diarylbutadiene compound(s) are present in proportions ranging from 0.1% to 20% by weight, more preferably from 1 to 10% by weight relative to the total weight of the emulsion.

18. Emulsion according to any one of Claims 1 to 17, **characterized in that** it additionally contains at least one additional organic sunscreening agent active in UV-A and/or UV-B, water-soluble, fat-soluble or insoluble in the commonly used cosmetic solvents.

19. Emulsion according to Claim 18, where the additional organic screening agents are chosen from anthranilates; cinnamic derivatives; dibenzoylmethane derivatives; salicylic derivatives, camphor derivatives; triazine derivatives; benzophenone derivatives; β, β' - diphenyl acrylate derivatives; benzotriazole derivatives; benzalmalonate derivatives; benzimidazole derivatives; imidazolines; bis-benzoazolyl derivatives; p-aminobenzoic acid (PABA) derivatives; benzoxazole derivatives; methylenebis(hydroxyphenylbenzotriazole) derivatives; screening polymers and screening silicones; dimers derived from α-alkylstyrene and mixtures thereof.

20. Emulsion according to Claim 19, where the additional organic screening agents are chosen from
Ethylhexyl Salicylate,
Ethylhexyl Methoxycinnamate,
Octocrylene,
Butyl Methoxydibenzoylmethane,
Phenylbenzimidazole Sulphonic Acid,
Benzophenone-3,
Benzophenone-4,
Benzophenone-5,
n-Hexyl 2-(4-diethylamino-2-hydroxybenzoyl) benzoate,
4-Methylbenzylidene camphor,
Terephthalylidene Dicamphor Sulphonic acid,
Disodium Phenyl Dibenzimidazole Tetra-sulphonate,
2,4,6-Tris(4'-diisobutyl aminobenzalmalonate)S-triazine
Anisotriazine,
Ethylhexyl triazone,
Diethylhexyl Butamido Triazone,
Methylene bis-Benzotriazolyl Tetramethylbutyl phenol,
Drometrizole Trisiloxane,

Polysilicone 15,
2,4-Bis-[5-1-(dimethylpropyl)benzoxazol-2-yl-(4-phenyl)imino]-6-(2-ethylhexyl)imino-1,3,5-triazine
and mixtures thereof.

**21.** Emulsion according to any one of Claims 1 to 20, **characterized in that** it additionally contains at least one agent for artificial bronzing and/or tanning of the skin.

**22.** Emulsion according to any one of Claims 1 to 21, **characterized in that** it additionally contains at least one cosmetic adjuvant chosen from organic solvents, ionic or nonionic thickeners, demulcents, humectants, opacifying agents, stabilizers, emollients, silicones, insect repellents, perfumes, preservatives, surfactants, fillers, pigments, polymers, propellants, alkanizing or acidifying agents or any other ingredient customarily used in the cosmetic and/or dermatological field.

**23.** Emulsion according to any one of Claims 1 to 22, **characterized in that** it contains an emulsifying system.

**24.** Emulsion according to Claim 23, where the emulsifying system comprises one or more emulsifying agents of the nonionic type and chosen from polyoxyethylenated and/or polyoxypropylenated fatty alcohols, and optionally polyoxyethylenated and/or polyoxypropylenated fatty acid esters and polyol esters.

**25.** Emulsion according to Claim 24, where the emulsifying system has an overall HLB ranging from 9.5 to 11.5 approximately and preferably close to 10.

**26.** Emulsion according to Claim 25, **characterized in that** the content of emulsifying agent(s) is between 0.5 and 40% by weight, preferably between 2 and 10% by weight, relative to the total weight of the emulsion.

**27.** Emulsion according to any one of Claims 1 to 26, **characterized in that** the content by weight of aqueous phase represents from 50 to 95%, preferably from 70 to 90%, of the total weight of the emulsion.

**28.** Emulsion according to any one of Claims 1 to 27, **characterized in that** the content by weight of the oily phase represents from 5 to 50%, preferably from 10 to 30%, of the total weight of the composition.

**29.** Method of preparing an oil-in-water emulsion as defined in any one of Claims 1 to 28, **characterized in that** it comprises the following steps:

(i) A fatty phase, on the one hand, and an aqueous phase, on the other hand, are mixed in the presence of a suitable emulsifying system and of at least one UV-A-screening agent of the 4,4-diarylbutadiene type, with stirring, the said mixing being carried out at a temperature greater than the phase inversion temperature (PIT) of the medium, so as to obtain a water-in-oil type emulsion,
(ii) the temperature of the emulsion thus obtained is brought to a temperature below the said phase inversion temperature, thereby obtaining an ultrafine emulsion of the oil-in-water type,
(iii) inorganic nanopigments are introduced during the carrying out of step (i) and/or at the end of step (ii).

**30.** Use of an emulsion defined in any one of Claims 1 to 28, for the manufacture of a composition for the cosmetic treatment of the skin, the lips and the hair and the scalp.

**31.** Use of an emulsion defined in any one of Claims 1 to 28, for the manufacture of a product for the protection and/or care of the skin, the lips and/or the hair and/or for making up the skin and/or the lips.

**32.** Use of a UV-A- screening agent of the 4,4-diarylbutadiene type as defined in Claims 1 to 15, in an oil-in-water emulsion capable of being obtained according to the phase inversion technique, in which the average size of the globules which constitute the oily phase of the said emulsion is between 100 nm and 1 000 nm and comprising inorganic nanopigments based on metal oxides and at least one organic UV-screening agent, with the aim of improving the stability to water of the sun protection factor.

## Patentansprüche

**1.** Öl-in-Wasser-Emulsion, die durch Phaseninversion erhältlich ist, bei der die mittlere Größe der Kügelchen, die die

Ölphase der Emulsion bilden, im Bereich von 100 bis 1000 nm liegt und die anorganische Nanopigmente auf der Basis von Metalloxiden und mindestens ein organisches UV-Filter enthält, **dadurch gekennzeichnet, dass** sie mindestens ein organisches UV-A-Filter vom 4,4-Diarylbutadientyp enthält.

2. Emulsion nach Anspruch 1, **dadurch gekennzeichnet, dass** die mittlere Größe der Ölkügelchen im Bereich von 100 bis 500 nm liegt.

3. Emulsion nach einem der Ansprüche 1 oder 2, **dadurch gekennzeichnet, dass** mindestens 90 % der Anzahl der Kügelchen eine Größe im Bereich von 100 bis 1000 nm und vorzugsweise im Bereich von 100 bis 500 nm aufweist.

4. Emulsion nach einem der Ansprüche 1 bis 3, **dadurch gekennzeichnet, dass** die Nanopigmente unter den umhüllten oder nichtumhüllten Oxiden von Titan, Zink, Eisen, Zirconium, Cer und deren Gemischen ausgewählt ist.

5. Emulsion nach Anspruch 4, **dadurch gekennzeichnet, dass** die Nanopigmente auf umhülltem oder nichtumhülltem Titanoxid basieren.

6. Emulsion nach Anspruch 5, **dadurch gekennzeichnet, dass** das Titanoxid in Form von Rutil, in Form von Anatas oder amorph vorliegt.

7. Emulsion nach einem der Ansprüche 1 bis 6, **dadurch gekennzeichnet, dass** das Mengenanteil der Nanopigmente 0,5 bis 40 Gew.-% und vorzugsweise 1 bis 30 Gew.-% des Gesamtgewichts der Zusammensetzung ausmacht.

8. Emulsion nach einem der Ansprüche 1 bis 7, wobei das UV-A-Filter vom 4,4-Diarylbutadientyp der folgenden Formel (I) entspricht:

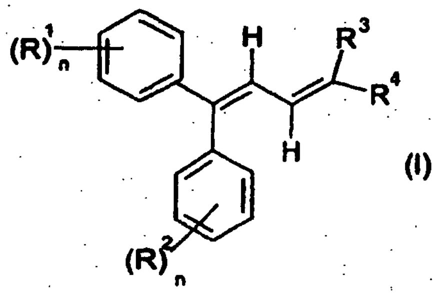

(I)

in der das Dien-System die Konfiguration Z,Z; Z,E; E,Z oder E,E oder Mischungen dieser Konfigurationen aufweist und in der bedeuten:

- $R^1$ und $R^2$, die gleich oder verschieden sind, Wasserstoff, eine $C_{1-20}$-Alkylgruppe; eine $C_{2-10}$-Alkenylgruppe; eine $C_{1-12}$-Alkoxygruppe; eine $C_{3-10}$-Cycloalkylgruppe; eine $C_{3-10}$-Cycloalkenylgruppe; eine $C_{1-20}$-Alkoxycarbonylgruppe; eine $C_{1-12}$-Monoalkylaminogruppe; eine $C_{1-12}$-Dialkylaminogruppe; Aryl; Heteroaryl oder einen Wasserlöslichkeit vermittelnden Substituenten, der unter Carboxylat, Sulfonat oder Ammonium ausgewählt ist;
- $R^3$ eine Gruppe $COOR^5$; $COR^5$; $CONR^5R^6$; CN; $O=S(-R^5)=O$; $O=S(-OR^5)=O$; $R^7O-P-(-OR^8)=O$; eine $C_{1-20}$-Alkylgruppe; eine $C_{2-10}$-Alkenylgruppe; eine $C_{3-10}$-Cycloalkylgruppe; eine $C_{7-10}$-Bicycloalkylgruppe; eine $C_{3-10}$-Cycloalkenylgruppe; eine $C_{7-10}$-Bicycloalkenylgruppe; eine $C_{6-18}$-Arylgruppe, die gegebenenfalls substituiert ist; eine $C_{3-7}$-Heteroarylgruppe, die gegebenenfalls substituiert ist;
- $R^4$ eine Gruppe $COOR^6$; $COR^6$; $CONR^5R^6$; CN; $O=S(-R^6)=O$; $O=S(-OR^6)=O$; $R^7O-P-(-OR^8)=O$; eine $C_{1-20}$-Alkylgruppe; eine $C_{2-10}$-Alkenylgruppe; eine $C_{3-10}$-Cycloalkylgruppe; eine $C_{7-10}$-Bicycloalkylgruppe; eine $C_{3-10}$-Cycloalkenylgruppe; eine $C_{7-10}$-Bicycloalkenylgruppe; eine $C_{6-18}$-Arylgruppe, die gegebenenfalls substituiert ist; eine $C_{3-7}$-Heteroarylgruppe, die gegebenenfalls substituiert ist;
- $R^5$ bis $R^8$, die gleich oder verschieden sind, Wasserstoff; eine $C_{1-20}$-Alkylgruppe; eine $C_{2-10}$-Alkenylgruppe; eine $C_{3-10}$-Cycloalkylgruppe; eine $C_{7-10}$-Bicycloalkylgruppe; eine $C_{3-10}$-Bicycloalkenylgruppe; eine $C_{7-10}$-Cycloalkenylgruppe; eine Arylgruppe, die gegebenenfalls substituiert ist; eine Heteroarylgruppe, die gegebenenfalls substituiert ist;
- n 1 bis 3;

wobei die Gruppen $R^3$ bis $R^8$ mit den Kohlenstoffatomen, an die sie verbunden sind, einen $C_{5-6}$-Ring bilden können, der kondensiert sein kann.

**9.** Emulsion nach Anspruch 8, wobei die Verbindung der Formel (I) unter den Verbindungen ausgewählt ist, für die bedeuten:

- n = 1 oder 2;
- $R^1$ und $R^2$, die gleich oder verschieden sind, Wasserstoff, eine $C_{1-20}$-Alkylgruppe; eine $C_{1-12}$-Alkoxygruppe; eine $C_{1-12}$-Monoalkylaminogruppe, eine $C_{1-12}$-Dialkylaminogruppe; einen Wasserlöslichkeit vermittelnden Substituenten, der unter Carboxylat, Sulfonat oder Ammonium ausgewählt ist;
- $R^3$ eine Gruppe $COOR^5$; $COR^5$; $CONR^5R^6$; eine $C_{1-20}$-Alkylgruppe; eine $C_{3-10}$-Cycloalkylgruppe; eine $C_{3-10}$-Cycloalkenylgruppe; eine $C_{7-10}$-Bicycloalkylgruppe; Phenyl, Naphthyl oder Thienyl, die gegebenenfalls substituiert sind;
- $R^4$ eine Gruppe $COOR^6$; $COR^6$; $CONR^5R^6$; eine $C_{1-20}$-Alkylgruppe; eine $C_{3-6}$-Cycloalkylgruppe; eine $C_{3-10}$-Cycloalkenylgruppe; eine $C_{7-10}$-Bicycloalkylgruppe; Phenyl, Naphthyl oder Thienyl, die gegebenenfalls substituiert sind;
- $R^5$ und $R^6$, die gleich oder verschieden sind, Wasserstoff; eine $C_{1-12}$-Alkylgruppe; eine $C_{3-10}$-Cycloalkylgruppe; eine $C_{3-10}$-Cycloalkenylgruppe; eine $C_{7-10}$-Bicycloalkylgruppe; eine $C_{3-10}$-Bicycloalkenylgruppe; Phenyl oder Naphthyl, die gegebenenfalls substituiert sind.

**10.** Emulsion nach Anspruch 9, wobei die Verbindung der Formel (I) unter den Verbindungen ausgewählt ist, für die bedeuten:

- $R^1$ und $R^2$, die gleich oder verschieden sind, Wasserstoff, eine $C_{1-20}$-Alkylgruppe; eine $C_{1-20}$-Alkoxygruppe; einen Wasserlöslichkeit vermittelnden Substituenten, der unter Carboxylat, Sulfonat oder Ammonium ausgewählt ist;
- $R^3$ eine Gruppe $COOR^5$; $COR^5$; $CONR^5R^6$;
- $R^4$ eine Gruppe $COOR^6$; $COR^6$; $CONR^5R^6$;
- $R^5$ und $R^6$, die gleich oder verschieden sind, Wasserstoff; eine $C_{1-12}$-Alkylgruppe; eine $C_{3-6}$-Cycloalkylgruppe; eine $C_{3-10}$-Cycloalkenylgruppe; eine $C_{7-10}$-Bicycloalkylgruppe; eine $C_{3-10}$-Bicycloalkenylgruppe; Phenyl oder Naphthyl, die gegebenenfalls substituiert sind.

**11.** Emulsion nach Anspruch 10, wobei die Verbindung der Formel (I) unter den Verbindungen der folgenden Formel (I') ausgewählt ist:

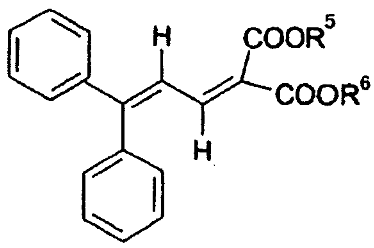

(I')

wobei $R^5$ und $R^6$, die gleich oder verschieden sind, Wasserstoff; eine $C_{1-20}$-Alkylgruppe; eine $C_{3-6}$-Cycloalkylgruppe; eine $C_{3-10}$-Cycloalklenylgruppe bedeuten.

**12.** Emulsion nach Anspruch 11, wobei die Verbindung der Formel (I') das 1,1-Dicarboxy-(2',2'-dimethyl-propyl)-4,4-diphenylbutadien der folgenden Struktur ist:

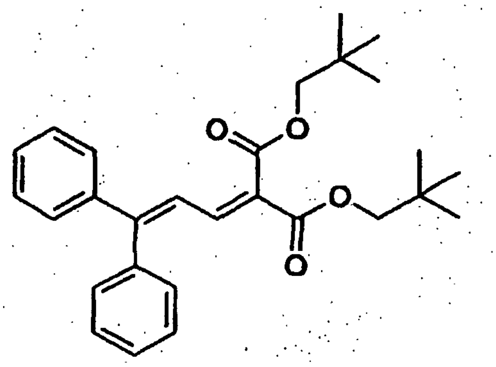

(Verbindung f).

13. Emulsion nach einem der Ansprüche 1 bis 7, wobei das UV-A-Filter vom 4,4-Diarylbutadientyp der folgenden Formel (II) entspricht:

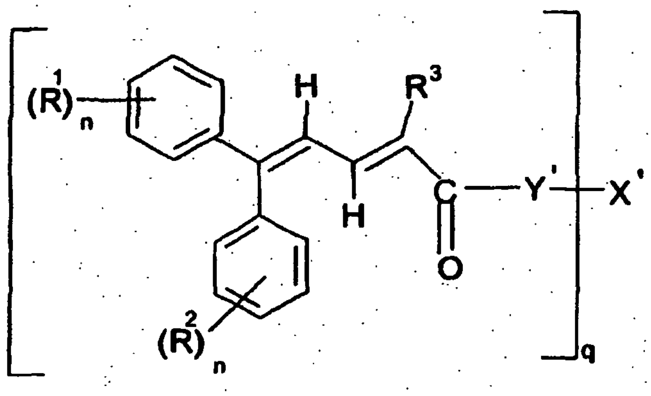

in der das Dien-System die Konfiguration Z,Z; Z,E; E,Z oder E,E oder Mischungen dieser Konfigurationen hat und worin bedeuten:

- $R^1$, $R^2$, $R^3$ und n die in Anspruch 8 für die Formel (I) angegebenen Bedeutungen;
- Y' eine Gruppe -O- oder -$NR^9$-;
- $R^9$ Wasserstoff; eine geradkettige oder verzweigte $C_{1-20}$-Alkylgruppe; eine $C_{2-10}$-Alkenylgruppe; eine $C_{3-10}$-Cycloalkylgruppe; eine $C_{7-10}$-Bicycloalkylgruppe; eine $C_{3-10}$-Cycloalkenylgruppe; eine $C_{7-10}$-Bicycloalkenylgruppe; Aryl; Heteroaryl;
- X' einen geradkettigen oder verzweigten, aliphatischen oder cycloaliphatischen $C_{2-20}$-Polyolrest, der 2 bis 10 Hydroxygruppen und die Valenz q aufweist; wobei die Kohlenstoffkette dieses Restes unterbrochen sein kann durch ein oder mehrere Schwefel- oder Sauerstoffatome; eine oder mehrere Imingruppen; eine oder mehrere $C_{1-4}$-Alkyliminogruppen;
- q 2 bis 10.

14. Emulsion nach Anspruch 13, wobei die Verbindung der Formel (II) unter den Verbindungen ausgewählt ist, für die bedeuten:

- $R^1$ und $R^2$, die gleich oder verschieden sind, Wasserstoff, eine $C_{1-12}$-Alkylgruppe; eine $C_{1-8}$-Alkoxygruppe; einen Wasserlöslichkeit vermittelnden Substituenten, der unter Carboxylat, Sulfonat oder Ammonium ausgewählt ist;
- $R^3$ eine Gruppe $COOR^5$; $CONR^5R^6$; CN; eine $C_{3-10}$-Cycloalkylgruppe; eine $C_{7-10}$-Bicycloalkylgruppe;
- $R^5$ und $R^6$, die gleich oder verschieden sind, eine geradkettige oder verzweigte $C_{1-20}$-Alkylgruppe; eine $C_{3-10}$-Cycloalkylgruppe; eine $C_{7-10}$-Bicycloalkylgruppe; Naphthyl oder Phenyl, die gegebenenfalls substituiert sind;
- X' einen $C_2$-$_{20}$-Polyolrest, der 2 bis 6 Hydroxygruppen und insbesondere 2 bis 4 Hydroxygruppen enthält.

15. Emulsion nach Anspruch 14, wobei die Verbindung der Formel (II) unter den Verbindungen ausgewählt ist, für die X' einen E-thanolrest oder Pentaerythritolrest bedeutet.

16. Emulsion nach Anspruch 15, wobei die Verbindung der Formel (II) unter den folgenden Verbindungen ausgewählt ist:

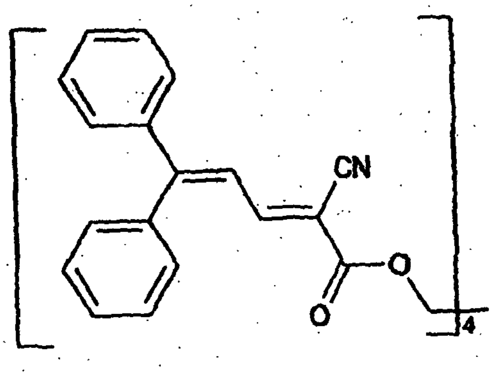

(Verbindung g)

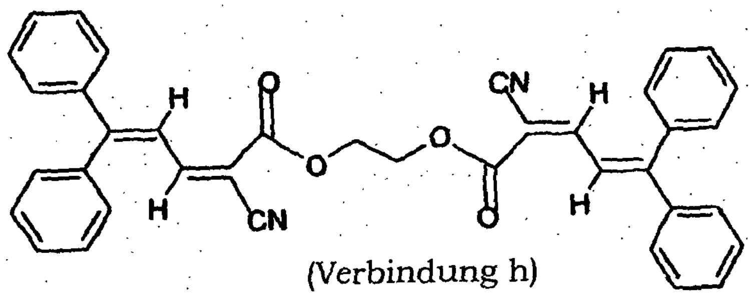

(Verbindung h)

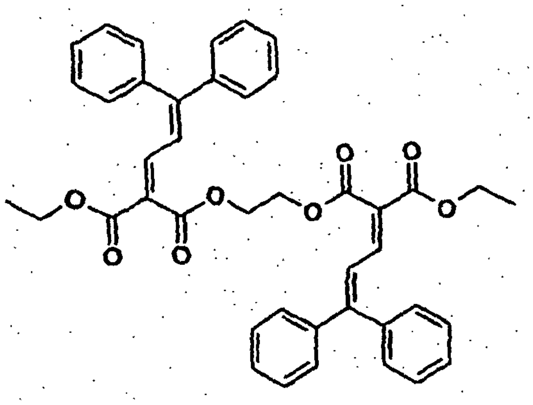

(Verbindung i).

17. Emulsion nach einem der Ansprüche 1 bis 16, **dadurch gekennzeichnet, dass** das oder die 4,4-Diarylbutadien (e) in einem Anteil von 0,1 bis 20 Gew.-% und vorzugsweise 1 bis 10 Gew.-%, bezogen auf das Gesamtgewicht

der Emulsion, enthalten sind.

18. Emulsion nach einem der Ansprüche 1 bis 17, **dadurch gekennzeichnet, dass** sie darüber hinaus mindestens ein ergänzendes, organisches Sonnenschutzfilter umfasst, das im UV-A- und/oder UV-B-Bereich wirksam und wasserlöslich, fettlöslich oder in den üblicherweise verwendeten kosmetischen Lösungsmitteln unlöslich ist.

19. Emulsion nach Anspruch 18, wobei die ergänzenden organischen Filter unter den Anthranilaten; Zimtsäurederivaten; Dibenzoylmethanderivaten; Salicylsäurederivaten, Campherderivaten; Triazinderivaten; Benzophenonderivaten; β, β-Diphenylacrylatderivaten; Benzotriazolderivaten; Benzalmalonatderivaten; Benzimidazolderivaten; Imidazolinen; Bis-benzoazolylderivaten; Derivaten von p-Aminobenzoesäure (PABA); Benzoxazolderivaten; Derivaten von Methylen-bis(hydroxyphenyl-benzotriazol); Polymerfiltern und Siliconfiltern; dimeren Derivaten von α-Alkylstyrol und deren Gemischen ausgewählt sind.

20. Emulsion nach Anspruch 19, wobei die ergänzenden organischen Filter ausgewählt sind unter:

Ethylhexyl Salicylate,
Ethylhexyl Methoxycinnamate,
Octocrylene,
Butyl Methoxydibenzoylmethane,
Phenylbenzimidazole Sulfonic Acid,
Benzophenone-3,
Benzophenone-4,
Benzophenone-5,
n-Hexyl-2-(4-diethylamino-2-hydroxybenzoyl)-benzoat,
4-Methylbenzylidene Camphor,
Terephthalylidene Dicamphor Sulfonic Acid,
Disodium Phenyl Dibenzimidazole Tetrasulfonate,
2,4,6-Tris-(diisobutyl-4'amino-benzalmalonat)-s-triazin,
Anisotriazine,
Ethylhexyl Triazone,
Diethylhexyl Butamido Triazone,
Methylene bis-Benzotriazolyl Tetramethylbutylphenol,
Drometrizole Trisiloxane,
Polysilicone-15,
2,4-Bis[5-1-(dimethylpropyl)benzoxazol-2-yl-(4-phenyl)-imino]-6-(2-ethylhexyl)-imino-1,3,5-triazin und ihren Gemischen.

21. Emulsion nach einem der Ansprüche 1 bis 20, **dadurch gekennzeichnet, dass** sie ferner mindestens ein Bräunungsmittel und/oder Mittel zur künstlichen Braunfärbung der Haut enthält.

22. Emulsion nach einem der Ansprüche 1 bis 21, **dadurch gekennzeichnet, dass** sie ferner mindestens einen kosmetischen Zusatzstoff enthält, der unter den organischen Lösungsmitteln, ionischen oder nichtionischen Verdikkungsmitteln, beruhigenden Stoffen, Feuchthaltemitteln, Trübungsmitteln, Stabilisatoren, Emollientien, Siliconen, insektenabwehrenden Wirkstoffen, Parfums, Konservierungsmitteln, grenzflächenaktiven Stoffen, Füllstoffen, Pigmenten, Polymeren, Treibmitteln, Alkalisierungsmitteln oder Ansäuerungsmitteln oder beliebigen anderen Bestandteilen, die gewöhnlich auf dem Gebiet der Kosmetik und/oder Dermatologie verwendet werden, ausgewählt ist.

23. Emulsion nach einem der Ansprüche 1 bis 22, **dadurch gekennzeichnet, dass** sie ein Emulgatorsystem enthält.

24. Emulsion nach Anspruch 23, wobei das Emulgatorsystem einen oder mehrere Emulgatoren vom nichtionischen Typ enthält, die unter den polyethoxylierten und/oder polypropoxylierten Fettalkoholen und Estern von Fettsäuren und Polyolen, die gegebenenfalls polyethoxyliert und/oder polypropoxyliert sind, ausgewählt sind.

25. Emulsion nach Anspruch 24, wobei das Emulgatorsystem einen Gesamt-HLB-Wert von etwa 9,5 bis 11,5 und vorzugsweise in der Gegend von 10 aufweist.

26. Emulsion nach Anspruch 25, wobei der Anteil des Emulgators oder der Emulgatoren im Bereich von 0,5 bis 40 Gew.-% und vorzugsweise 2 bis 10 Gew.-%, bezogen auf das Gesamtgewicht der Zusammensetzung, liegt.

**27.** Emulsion nach einem der Ansprüche 1 bis 26, **dadurch gekennzeichnet, dass** der Gewichtsanteil der wässrigen Phase 50 bis 95 % und vorzugsweise 70 bis 90 % des Gesamtgewichts der Zusammensetzung ausmacht.

**28.** Emulsion nach einem der Ansprüche 1 bis 27, **dadurch gekennzeichnet, dass** der Gewichtsanteil der Ölphase 5 bis 50 % und vorzugsweise 10 bis 30 % des Gesamtgewichts der Zusammensetzung ausmacht.

**29.** Verfahren zur Herstellung einer Öl-in-Wasser-Emulsion nach einem der Ansprüche 1 bis 28, **dadurch gekennzeichnet, dass** es die folgenden Schritte umfasst:

(i) man vermischt in Gegenwart eines geeigneten Emulgatorsystems und mindestens eines UV-A-Filters von 4,4-Diarylbutadientyp unter Rühren eine wässrige Phase und eine Ölphase, wobei das Mischen bei einer Temperatur über der Phaseninversionstemperatur (PIT) des Mediums durchgeführt wird, um eine Wasser-in-Öl-Emulsion zu bilden;
(ii) die Temperatur der erhaltenen Emulsion wird auf eine Temperatur unter der Phaseninversionstemperatur gebracht, wodurch eine ultrafeine Emulsion vom Öl-in-Wasser-Typ gebildet wird,
(iii) die anorganischen Nanopigmente werden in Schritt (i) und/oder in Schritt (ii) eingearbeitet.

**30.** Verwendung einer Emulsion nach einem der Ansprüche 1 bis 28 zur Herstellung einer Zusammensetzung für die kosmetische Behandlung der Haut, der Lippen, der Haare und der Kopfhaut.

**31.** Verwendung einer Emulsion nach einem der Ansprüche 1 bis 28 zur Herstellung eines Produktes für den Schutz und/oder die Pflege der Haut, der Lippen und/oder der Haare und/oder zum Schminken der Haut und/oder der Lippen.

**32.** Verwendung eines UV-A-Filters vom 4,4-Diarylbutadientyp, wie er in einem der Ansprüche 1 bis 15 definiert ist, in einer Öl-in-Wasser-Emulsion, die durch Phaseninversion erhältlich ist, bei der die mittlere Größe der Kügelchen, die die Ölphase der Emulsion bilden, im Bereich von 100 bis 1000 nm liegt und die anorganische Nanopigmente auf der Basis von Metalloxiden und mindestens ein organisches UV-Filter enthält, um die Remanenz des Sonnenschutzfaktor zu verbessern.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2528420 A
- FR 2639347 A
- DE 19755649
- EP 916335 A
- EP 1133980 A
- EP 1133981 A
- EP 1008586 A
- US 4367390 A
- EP 863145 A
- EP 517104 A
- EP 570838 A
- EP 796851 A
- EP 775698 A
- EP 878469 A
- EP 933376 A
- EP 507691 A
- EP 507692 A
- EP 790243 A
- EP 944624 A
- EP 669323 A
- US 2463264 A
- EP 0832642 A
- EP 1027883 A
- EP 1300137 A
- DE 10162844
- US 5237071 A
- US 5166355 A
- GB 2303549 A
- DE 19726184
- EP 893119 A
- WO 9304665 A
- DE 19855649
- FR 2466492
- WO 9735842 A
- EP 903342 A
- WO 9725970 A
- US 4077441 A
- US 4850517 A


### Littérature non-brevet citée dans la description


- *Cosmetics & Toiletries,* Février 1990, vol. 105, 53-64
- **TH FÖRSTER et al.** Phase Inversion Emusification. *Cosmetics & Toiletries,* Décembre 1991, vol. 106, 49-52
- *J. Soc. Cosm. Chem.,* 1954, vol. 5, 249-256
- **B. L. DIFFEY et al.** *J. Soc. Cosmet.,* 1989, vol. 40, 127-133